# EUROPEAN PATENT APPLICATION

(11) **EP 4 086 345 A1**
(43) Date of publication of application: **09.11.2022**
(21) Application number: 20908810.3
(22) Date of filing: 30.12.2020
(51) Int. Cl.: C12N 15/113, C12N 15/11, C12N 15/10, A61K 31/712

(54) **LEAPER TECHNOLOGY BASED METHOD FOR TREATING MPS IH AND COMPOSITION**

(30) Priority: 30.12.2019 WO PCT/CN2019/129952
(71) Applicant: EdiGene Therapeutics (Beijing) Inc., Beijing 102206 (CN)
(72) Inventor: YUAN, Pengfei, Beijing 102206 (CN); ZHAO, Yanxia, Beijing 102206 (CN); LIU, Nengyin, Beijing 102206 (CN); YI, Zexuan, Beijing 102206 (CN); TANG, Gangbin, Beijing 102206 (CN)
(74) Representative: Lavoix
(86) International application number: PCT/CN2020/141506
(87) International publication number: WO 2021/136408

(57) **Abstract**

The present application relates to an LEAPER-based method for targeted editing of RNA, which comprises: safely and effectively performing adenosine-to-inosine RNA editing (A-to-I RNA editing) by LEAPER in vivo, thereby the pathogenic mutation sites can be accurately repaired, and all diseases caused by G>A mutations, such as MPS IH, can be treated.

## Description

### FIELD OF THE INVENTION

The present application relates to the field of gene editing therapeutics, and specifically, relates to a method for treating MPS IH by LEAPER (Leveraging Endogenous ADAR for Programmable Editing on RNA)-based targeted editing of RNA, which comprises: performing site-directed A-to-I RNA editing by LEAPER in vivo to treat diseases caused by G>A mutations, such as MPS IH.

### BACKGROUND OF THE INVENTION

Hurler syndrome, also known as mucopolysaccharidoses IH (MPS IH) or glycosaminoglycan disorder IH, is the most severe one among three subtypes, i.e. IH, IH/S, and IS, of MPSI. It is a disabling and fatal genetic metabolic disease caused by a deficiency of α-L-iduronidase (IDUA) in patients with this disease, and is an autosomal recessive (AR) disorder. The underlying mechanism of Hurler syndrome is mutations in the IDUA gene for encoding the IDUA protein located at 4p16.3 of chromosome 4. So far, more than 200 pathogenic IDUA mutations are known, and the most common type is a G-to-A mutation at the 1205 site of cDNA. Due to this mutation, the original tryptophan is converted into a termination codon, whereby a protein obtained by translation lacks all amino acids (NM_000203.4(IDUA)-c.1205G-A (p.Trp402Ter)) after this site and loses the IDUA enzymatic activity. This mutation type can cause 63% of the total incidence (Worldwide distribution of common IDUA pathogenic variants, Poletto, Edina (2018). Clinical Genetics. 94. 10.1111/cge.13224.). α-L-iduronidase is responsible for the degradation of glycosaminoglycans (GAGs) in the lysosomes of cells. Symptoms vary between the patients with Hurler syndrome, and they may not be present at birth. The earliest symptom that begins at 3-6 months of age is coarsening of the facial features, then other symptoms including prominent frontal bones, skeletal abnormalities, growth stopping, and limited language capabilities will occur, and death usually occurs by age 10.

There is no cure for Hurler syndrome. Two treatments have been currently approved: enzyme replacement therapy (ERT) and hematopoietic stem cell transplantation (HSCT). ERT has shown good effects in terms of visceral phenotype, including reduction in liver size, improvement in respiratory function, and overall improvement in patient mobility. On the downside, it cannot reach the central nervous system and therefore cannot prevent cognitive impairment. Successful HSCT can prevent most clinical symptoms, including neurological symptoms. However, it must be applied for treatment before clinical symptoms appear (preferably before 8 months of age), and only applicable to patients with severe diseases due to its high mortality (Combination of enzyme replacement and hematopoietic stem cell transplantation as therapy for Hurler Syndrome. Tolar, J (2008). Bone marrow transplantation. 41. 531-5. 10.1038/sj.bmt.1705934).

At present, the principle of gene editing technique for treating Hurler syndrome is to use a zinc finger nuclease (ZFN) and an adeno-associated virus (AAV) to insert a cDNA sequence for encoding normal IDUA proteins into the genome of hepatocytes, but this method still cannot eliminate brain and skeletal system symptoms of Hurler syndrome, and the off-target caused by DNA editing is a high concern.

In theory, CRISPR (Clustered regularly interspaced short palindromic repeats), which has been developing rapidly in recent years, can also be used to treat Hurler syndrome. Many researchers and biotech companies are also committed to applying this technique to the clinic. For example, in September 2019, results from clinical trials of editing stem cells by CRISPR and infusing them back into patients to treat AIDS and leukemia were reported for the first time, making a great contribution to the application of CRISPR in gene therapy. Although CRISPR has great potential applications, it also has a number of drawbacks, which make it difficult to be applied from the research phase to clinical treatment. One of the problems is the core enzyme used in CRISPR: Cas9. CRISPR-based DNA editing relies on exogenously expressed Cas9 or other nucleases with similar functions, which causes several problems. First, the nucleases to be exogenously expressed usually have large molecular weight, which causes dramatical reduction in efficiency of their delivery to the body via viral vectors. Second, the exogenous expression of nucleases makes them possible to have potential for nuclease off-target, which will make them potentially carcinogenic in application. Finally, the exogenously expressed nucleases are found in bacteria but not in humans or mammals, and possibly induce an immune response in a patient, which on the one hand may cause damage to the patient, and on the other hand may cause neutralization of the exogenously expressed nucleases, thus losing their proper activity or hindering further therapeutic intervention.

In 2017, Feng Zhang's group had reported an RNA editing technique called REPAIR (RNA Editing for Programmable A to I Replacement) (RNA editing with CRISPR-Cas13, Cox et al., 2017). This technique can also achieve targeted A-to-I RNA editing by exogenously expressed Cas13-ADAR fusion protein and single guide RNA (sgRNA). However, this technique, like CRISPR, still relies on the expression of the exogenous protein, and cannot solve the problems caused by the expression of the exogenous protein.

In January 2019, Thorsten Stafforst's group had reported a nucleic acid editing technique called RESTORE (recruiting endogenous ADAR to specific trans for oligonucleotide-mediated RNA editing, Merkle et al., 2019). This technique does not rely on exogenous proteins. However, on one hand, RESTORE can achieve high editing efficiency only in the presence of IFN-γ, which is a key factor in determining the development and severity of autoimmunity (Interferon-y and systemic autoimmunity, Pollard et al., 2013), thereby hindering application of this technique in the medical field. On the other hand, RESTORE also uses a guide RNA, which is a chemically synthesized oligonucleotide and needs to be artificially introduced with a large number of chemical modifications to ensure its stability.

In 2019, patent applications PCT/CN2019/110782 and PCT/CN2020/084922 had provided an engineering modified RNA. The RNA is partially complementary to a target transcript to recruit a natural ADAR1 or ADAR2 so as to convert adenosine to inosine at a specific site on the target RNA. The technique is called "LEAPER (Leveraging Endogenous ADAR for Programmable Editing on RNA)", and the RNA for recruiting ADAR can be called "dRNA" or "arRNA". The dRNA comprises a complementary RNA sequence that hybridizes to the target RNA, and can recruit adenosine deaminase acting on RNA (ADAR) to deaminate target adenosine (A) in the target RNA.

### SUMMARY OF THE INVENTION

In view of G-to-A mutations in the IDUA pathogenic gene that cause Hurler syndrome, especially the NM_000203.4(IDUA)-c.1205G-A (p.Trp402Ter) mutation that accounts for the highest proportion, the present application provides a novel technical solution for accurately editing a mutation site on the target RNA.

Specifically, the present application at least provides the following technical solutions.
1. A LEAPER-based method for target editing of a target RNA in a target cell, wherein the target RNA is an RNA comprising a G-to-A mutation in an IDUA gene transcript, and the method comprises:
   delivering a construct comprising an adenosine deaminase-recruiting RNA (arRNA) used for editing the target RNA or a construct for encoding the arRNA to the target cell, wherein the arRNA comprises a complementary RNA sequence that hybridizes with the target RNA, and can recruit adenosine deaminase acting on RNA (ADAR) to deaminate target adenosine (A) in the target RNA.
2. The method according to item 1, the arRNA introduces base C, A, U or G that is paired with the target A.

In some embodiments, the arRNA comprises a base C that is paired with the target A. In some embodiments, the arRNA comprises a base A that is paired with the target A. In some embodiments, the arRNA comprises a base U that is paired with the target A.
3. The method according to item 1 or 2, the length of the arRNA is about 151-61 nt, 131-66 nt, 121-66 nt, 111-66 nt, 91-66 nt or 81-66 nt. The present application discloses and covers any natural number within the numerical ranges.
4. The method according to item 3, wherein the distance between a targeting base in the arRNA and the 3'-terminal is 45-5 nt, 40-5 nt, 35-10 nt, 25-15 nt or 24-11 nt. The present application discloses and covers any natural number within the numerical ranges.
5. The method according to item 3 or 4, wherein the distance between the targeting base in the arRNA and the 5'-terminal is 80-30 nt, 70-35 nt, 60-40 nt, 55-35 nt or 55-45 nt. The present application discloses and covers any natural number within the numerical ranges.
6. The method according to any one of item 1 to 5, the target cell is a human cell.
7. The method according to any one of item 1 to 6, the target RNA is the RNA comprising the NM_000203.4(IDUA)-c.1205G-A (p.Trp402Ter) mutation site.
8. The method according to any one of item 1 to 7, the arRNA comprises any one of the following sequences: SEQ ID NO: 14, SEQ ID NO: 15, SEQ ID NO: 9, SEQ ID NO: 13, SEQ ID NO: 17, SEQ ID NO: 22, SEQ ID NO: 23, SEQ ID NO: 30, SEQ ID NO: 31, and SEQ ID NO: 34.
9. The method according to any one of item 1 to 5, wherein the arRNA comprises any one of the following sequences: SEQ ID NO: 44 and SEQ ID NO: 52.
10. The method according to any one of item 1 to 9, wherein the arRNA is chemically modified.
11. The method according to item 10, wherein the chemical modification comprises 2-O'-methylation (2'-OMe) or thiophosphate modification.
12. The method according to item 11, wherein the chemical modification is one or more selected from the group consisting of:
   2'-OMe modification on each of the first three nucleotides and the last three nucleotides in the arRNA sequence;
   thiophosphate bond linkage between nucleotides of the first three nucleotides and the last three nucleotides;
   2'-OMe modification on all bases U in the arRNA sequence;
   2'-OMe modification on the base A which is the 3' base nearest to the targeting base;
   2'-OMe modification on the base C which is the 5' base nearest to the targeting base;
   thiophosphate bond linkage between the targeting base with its 3' nearest base and 5'nearest base respectively;
   2'-OMe modification on each of the first five nucleotides and the last five nucleotides; and
   thiophosphate bond linkage between nucleotides of the first five nucleotides and the last five nucleotides.

In some specific embodiments, the chemical modification is selected from any one or more selected from the group consisting of:
CM1, which refers to that the first three nucleotides and the last three nucleotides in the arRNA sequence are individually subjected to 2'-OMe modification, the first three nucleotides and the last three nucleotides are linked via thiophosphate bonds between nucleotides, and meanwhile, all bases U in the arRNA sequence are subjected to 2'-OMe modification;
CM2, which refers to that the first three nucleotides and the last three nucleotides in the arRNA sequence are individually subjected to 2'-OMe modification, the first three nucleotides and the last three nucleotides are linked via thiophosphate bonds between nucleotides, and meanwhile, the 3' nearest base of the targeting base is base A subjected to 2'-OMe modification;
CM3, which refers to that the first three nucleotides and the last three nucleotides in the sequence are individually subjected to 2'-OMe modification, the first three nucleotides and the last three nucleotides are linked via thiophosphate bonds between nucleotides, and meanwhile, the 5' nearest base of the targeting base is base C subjected to 2'-OMe modification;
CM4, which refers to that the first three nucleotides and the last three nucleotides in the sequence are individually subjected to 2'-OMe modification, the first three nucleotides and the last three nucleotides are linked via thiophosphate bonds between nucleotides, and meanwhile, the targeting base is linked to the 3' nearest base and the 5' nearest base of the targeting base respectively via thiophosphate bonds; and
CM6, which refers to the first five nucleotides and the last five nucleotides in the sequence are individually subjected to 2'-OMe modification, and the first five nucleotides and the last five nucleotides are linked via thiophosphate bonds between nucleotides.
13. The method according to any one of item 1 to 9, the construct for encoding the arRNA is a linear nucleic acid chain, virus vector or plasmid.
14. The method according to item 13, the virus vector is an adeno-associated virus (AAV) vector or lentivirus expression vector.
15. The method according to any one of item 1 to 14, the delivery method is electrotransfection, lipofection, or lipid nanoparticle (LNP) delivery or infection.
16. The method according to item 15, the construct comprising the adenosine deaminase-recruiting RNA (arRNA) used for editing the target RNA or the construct for encoding the arRNA is delivered to the target cell via LNP.
17. The method according to any one of item 1 to 16, the delivery concentration of the arRNA is equal to or greater than 2.5 nM, 5 nM, 10 nM. 15 nM or 20 nM.

In the above embodiments, the target cell comprises hepatocyte or fibroblast.
18. An arRNA used for LEAPER-based targeted editing of target RNA in a target cell or a coding sequence of the arRNA, wherein the arRNA comprises or consists of any one of the following sequences: SEQ ID NO: 14, SEQ ID NO: 15, SEQ ID NO: 9, SEQ ID NO: 13, SEQ ID NO: 17, SEQ ID NO: 22, SEQ ID NO: 23, SEQ ID NO: 30, SEQ ID NO: 31, SEQ ID NO: 34, SEQ ID NO: 44, and SEQ ID NO: 52.
19. A plasmid, virus vector, liposome or lipid nanoparticle, which comprises the arRNA or the coding sequence of the arRNA of item 18.
20. A composition, preparation, kit or biological product, which comprises the arRNA or the coding sequence of the arRNA of item 18, or comprises the plasmid, virus vector, liposome or lipid nanoparticle of item 19.
21. The method for treating MPS IH in an individual, which comprises correcting a G-to-A mutation associated with MPS IH in a target cell of an individual by the method of any one of item 1 to 17.
22. The method according to item 20, the mutation is the NM_000203.4(IDUA)-c.1205G-A (p.Trp402Ter) mutation.
23. The method according to item 20 or 21, the arRNA is administrated at a frequency of ≥every 21 days, ≥17 every days, ≥every 14 days or ≥every 10 days.

In some embodiments, the present application also relates to use of any one of the following sequences in the preparation of a drug for treating MPS IH: SEQ ID NO: 14, SEQ ID NO: 15, SEQ ID NO: 9, SEQ ID NO: 13, SEQ ID NO: 17, SEQ ID NO: 22, SEQ ID NO: 23, SEQ ID NO: 30, SEQ ID NO: 31, SEQ ID NO: 34, SEQ ID NO: 44, and SEQ ID NO: 52.

The technical solutions of the present application can be used to safely and effectively perform A-to-I RNA editing in a target cell (e.g. hepatocyte and fibroblast.) in vivo, thereby accurately repairing a pathogenic mutation site such as the NM_000203.4(IDUA)-c.1205G-A (p.Trp402Ter) mutation, and recovering the normal expression of proteins encoded by RNA in vivo to achieve the purpose of treating MSP IH.

### BRIEF DESCRIPTION OF THE ACCOMPANYING DRAWINGS

Fig. 1 shows detection of the IDUA genotype of GM06214 cells.
Fig. 2 shows a test on cell electrotransfection.
Fig. 3A shows the design of the arRNAs specific to IDUA pre-mRNA and mRNA, and the detection of cellular function after editing; and Fig. 3B shows the design of the arRNAs specific to IDUA pre-mRNA and mRNA, and the detection of cellular editing efficiency.
Fig. 4A shows the design of an IDUA-reporter cell line; and Fig. 4B shows detection of the editing efficiency of arRNAs with different lengths (symmetrically truncated) on 293T-IDUA-Reporter.
Fig. 5A and Fig. 5B show the detection of the enzyme activity and the editing efficiency in GM06214 cells that are transfected with arRNAs with different lengths (symmetrically truncated) at different time points.
Fig. 6 shows the detection of the IDUA enzyme activity and the editing efficiency in GM06214 cells that are transfected with arRNAs (symmetrically truncated, truncated from the 3'-terminal, and truncated from the 5'-terminal) by lipofectmine RNAiMAX.

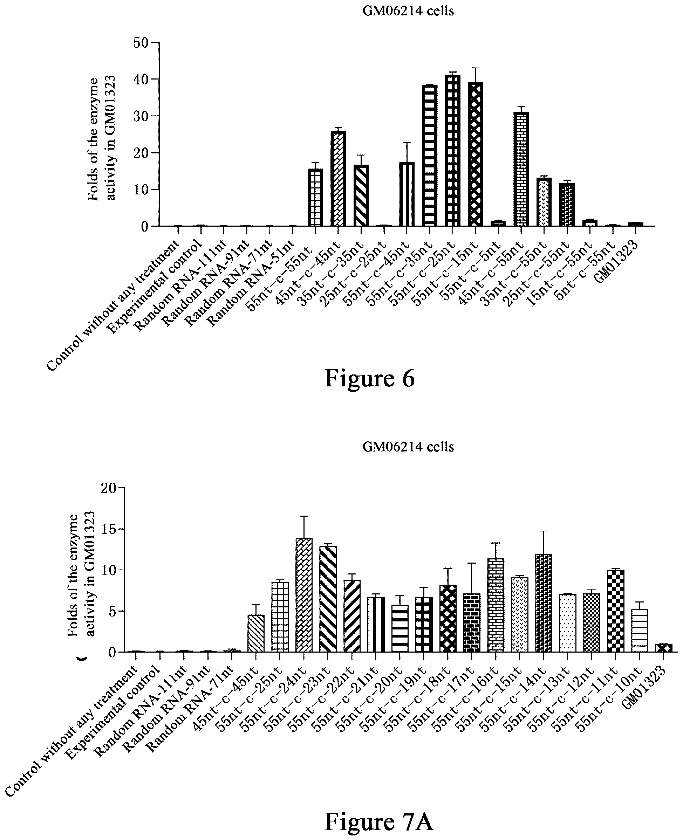

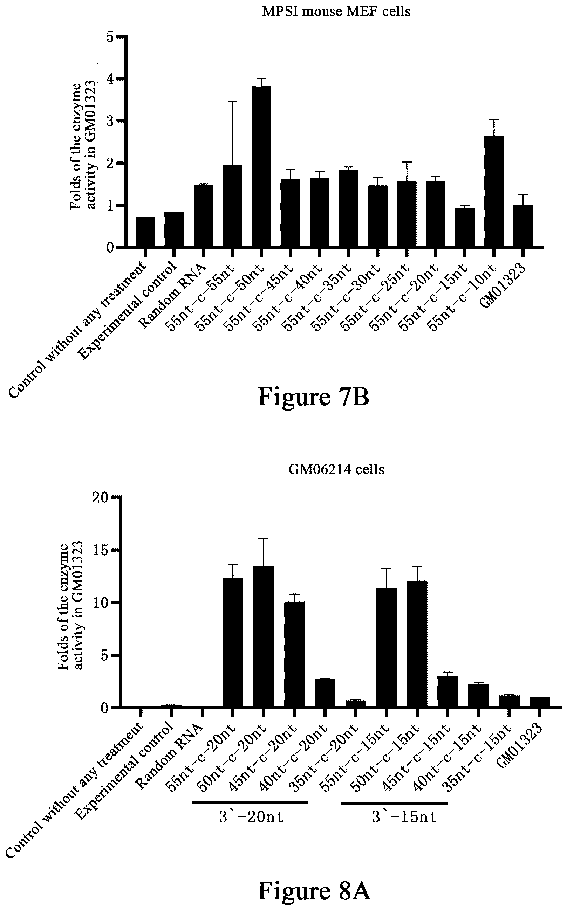

Fig. 7A shows the selection of the optimal length by detection of the enzyme activity in GM06214 cells that are transfected with arRNAs targeting a human IDUA mutation site, wherein the length of the arRNA is preferably 55nt-c-25nt to 55nt-c-10nt, that is, bases at the 3'-terminal are reduced one by one; and Fig. 7B shows the selection of the optimal length by detection of the enzyme activity in MSPI mouse MEF (MSPI mouse embryo fibroblast) cells that are transfected with arRNAs targeting a mouse IDUA mutation site, wherein the length of the arRNA is 55nt-c-55nt to 55nt-c-10nt, that is, bases at the 3'-terminal are reduced by every 5 bases.
Fig. 8A shows selection of arRNA with the optimal length by gradually truncating the length of the 5'-terminal at the conditions of two preferred lengths of the 3'-terminal; and Fig. 8B shows the influence of arRNAs, of which the length of the 3'-terminal is fixed at 14 nt and the length of the 5'-terminal is gradually truncated base by base, on the enzyme activity, wherein arRNA with the optimal length is screened out according to Fig. 8A and Fig. 8B.
Fig. 9 shows a comparison of the influence of different chemical modifications on the editing efficiency (reflected by the enzymatic activity) of arRNAs with 2 preferred lengths.
Fig. 10A to Fig. 10D show the editing efficiency of arRNAs targeting human and mouse IDUA mRNAs and the ability to produce functional IDUA proteins after editing under the conditions of the preferred length, the preferred chemical modification combination, and different concentrations of arRNA.
Fig. 11A to Fig. 11D show the IDUA enzyme activity in human or mouse cells at different time points after one-time transfection under the conditions of the preferred length and the preferred chemical modification combination.
Fig. 12A and Fig. 12B show the editing efficiency of arRNA targeting a site after the arRNA is delivered to primary human and mouse liver cells by different methods.
Fig. 13 shows the editing efficiency of arRNA targeting IDUA after the arRNA is delivered to human and mouse liver cells obtained by primary cell culture via LNP.
Fig 14 shows the IDUA editing efficiency in mouse liver cells 24 h after administration of a selected arRNA (SEQ ID NO: 52) targeting a mouse IDUA mutation that is prepared into LNP to a mouse by tail vein injection at different concentrations.

### DETAILED DESCRIPTION OF THE INVENTION

### Definition

RNA editing is a natural process occurring in eukaryotic cells. RNA editing involves the conversion of base A (adenosine) to base I (inosine) in RNA that occurs before protein translation and after DNA transcription, during translation, inosine is identified as G, and A-to-I RNA editing diversifies the transcriptome. The total number of RNAs will be increased several folds by accurately converting the RNA molecules. Such editing is catalyzed by adenosine deaminase acting on RNA (ADAR), so it is called site-directed RNA editing. The editing may occur in coding regions comprising intron and exon sequences, or may occur in non-coding regions, and the editing in coding regions can redefine protein-coding sequences.

As used herein, "LEAPER" is a technique using engineered RNA to recruit endogenous ADAR used for RNA editing, which has been disclosed in WO2020074001A1. As used herein, the engineered RNA refers to adenosine deaminase-recruiting RNA (arRNA), which can recruit ADAR or some compounds comprising an ADAR domain to deaminate target adenosine in target RNA.

As used herein, the term "adenosine deaminase acting on RNA (ADAR)" refers to a class of adenosine deaminase widely expressed in tissues of eukaryotes (including mammals such as humans), which can catalyze the conversion of adenosine (A) to inosine (I) in an RNA molecule. However, in the synthesis process of proteins in eukaryotes, I is usually translated into G.

As used herein, the "complementarity" of nucleic acids refers to the ability of a nucleic acid to form hydrogen bonds with another nucleic acid by the conventional Watson-Crick base pairing. The percentage of complementarity refers to the percentage of residues in a nucleic acid molecule that can form hydrogen bonds with residues in another nucleic acid molecule (i.e. Watson-Crick base pairing). For example, among 10 residues of a nucleic acid molecule, about 5, 6, 7, 8, 9 or 10 residues can form hydrogen bonds with residues in another nucleic acid molecule, which means the percentage of complementarity of the two nucleic acid molecules is about 50%, 60%, 70%, 80%, 90% or 100%. "Complete complementation" refers to that all continuous residues in a nucleotide sequence can form hydrogen bonds with the same number of continuous residues in another nucleotide sequence. As used herein, "substantially complementary" refers to that in about 40, 50, 60, 70, 80, 100, 150, 200, 250 or more nucleotide regions, the percentage of complementarity is at least about 70%, 75%, 80%, 85%, 90%, 95%, 97%, 98% or 99%, or refers to that two nucleic acids hybridize under strict conditions. For a single base or single nucleotide, the pairing of A with T or U, and pairing of C with G or I in Watson-Crick base pairing principle can be called complementation or matching, and vice versa. The pairing of the bases otherwise should be called non-complementation or non-matching.

"Hybridization" refers to a process in which one or more polynucleotides react to form a compound, and the compound is stabilized by hydrogen bonds between the bases of the nucleotide residues. The hydrogen bonds can also be formed by Watson Crick base pairing, Hoogstein pairing or any other sequence-specific methods. A sequence that can hybridize with a given sequence is called a "complementary sequence" of the given sequence.

As used herein, the term "electrotransfection" refers to electroporation transfection, by which cells are exposed to an electric field for microseconds to milliseconds, apertures or openings are temporarily formed on the cell membrane, and macromolecules, such as DNA, are delivered to the cells.

As used here, the term "lipofection (Lipo)" refers to a transfection technique using a liposome as a delivery vector in vivo or in vitro. Liposomes include neutral liposomes and cationic liposomes. For a neutral liposome, a macromolecule, such as nucleic acid, is wrapped with the lipid membrane, and then delivered to the cell membrane via the lipid membrane, and for a cationic liposome that is positively charged, a macromolecule to be delivered is not embedded in the cationic liposome in advance, the macromolecule that is negatively charged automatically binds to the positively charged liposome to form a macromolecule-cationic liposome compound, which will be adsorbed to the surface of the cell membrane that is negatively charged, and delivered to a cell by endocytosis.

As used herein, the term "lipid nanoparticle (LNP) delivery" refers to transmembrane delivery of a macromolecule, such as nucleic acid and protein, via a lipid nanoparticle. Lipid nanoparticles refer to particles synthesized by mixing two phases, which comprise an ethanol phase containing ionizable lipids, auxiliary phospholipids, cholesterol, and PEG lipids, and an acidic aqueous phase containing macromolecules such as nucleic acids and proteins. For example, RNA wrapped with LNP can be delivered to the cytoplasm by endocytosis.

Herein, the NM_000203.4(IDUA)-c.1205G-A (p.Trp402Ter) mutation refers to a G-to-A mutation at the 1205 site on the No. 000203.4 transcript of the IDUA gene, which will convert a coding sequence of tryptophan (Trp) on the 402 site of a peptide chain translated by the transcript to a termination codon (Ter), whereby the finally translated peptide chain lacks all amino acids after the 402 site and loses the IDUA enzymatic activity. Such a mutation will lead to a deficiency of active α-L-iduronidase, which affects the degradation of glycosaminoglycans in the lysosomes of cells, and eventually causes teratogenicity and even death of patients. However, the technical solutions of the present application can reverse the mutation during transcription to recover the IDUA enzymatic activity.

As used herein, the term "target RNA" refers to target RNA to be edited, which comprises adenosine (A) to be edited. The target RNA may be mature mRNA, or may be precursor mRNA. In the present application, the target RNA is more preferably precursor mRNA. Herein, a cell comprising the "target RNA" is called a target cell. The adenosine to be edited is called a "target base", "target adenosine" or "target A". In the present application, "target base", "target adenosine" and "target A" can be used interchangeably. A base, adjacent to target adenosine, at the 5'-terminal of target RNA is called a "5' adjacent base"; a base, adjacent to the target adenosine, at the 3'-terminal of the target RNA is called a "3' adjacent base"; and a base triplet composed of the target base, the 3' adjacent base, and the 5' adjacent base is called a "target base triplet" herein. When arRNA hybridizes with the target RNA, the base, corresponding to the target base, on the arRNA is called "targeting base"; the base, adjacent to the targeting base, at the 5'-terminal of the arRNA is called the "5' nearest base"; a base, adjacent to the targeting base, at the 3'-terminal of the arRNA is called the "3' nearest base", and a base triplet composed of the targeting base, the 3' nearest base, and the 5' nearest base is called a "targeting base triplet" herein.

As uses herein, the term "construct" refers to a nucleic acid vector comprising a certain nucleotide sequence, which may be a linear nucleic acid molecule, plasmid or virus vector. The nucleic acid molecule may be a single-stranded or double-stranded molecule. The nucleotide sequence may be a DNA sequence or RNA sequence. In some embodiments, the nucleotide sequence can work without being subjected to transcription, translation or expression. In some embodiments, the nucleotide sequence is a DNA sequence, and works in the form of RNA molecules that are generated by transcribing the nucleotide sequence. In some embodiments, the nucleotide sequence is an RNA sequence, and works in the form of polypeptides or proteins that are generated by translating the nucleotide sequence. In some embodiments, the nucleotide sequence is a DNA sequence, and work in the form of proteins generated by transcribing and translating the nucleotide sequence. The construct can be prepared into the form of virus, lipid nanoparticle or exosome, and then delivered to a target cell, or can be delivered to a target cell by electrotransformation, microjection or chemical conversion.

As used herein, the term "delivery" refers to introduction of biomacromolecules, such as nucleic acids and proteins, into the cell membrane by certain ways. The "delivery" methods include electrotransfection, lipofection, lipid nanoparticle delivery, virus delivery, and exosome delivery.

The term "modification" used in the present application refers to that one or more properties or functions of a nucleic acid or protein are changed by a chemical or biological method. For example, the composition or structure of the nucleic acid or protein is changed by gene engineering.

Unless otherwise define, all technical terms and scientific terms used herein have the same meanings as commonly understood by those of ordinary skill in the art to which the present application belongs.

### RNA editing method

The present application provides an LEAPER-based method for targeted editing of target RNA comprising a G-to-A mutation in the IDUA gene in the target cell, which comprises: a construct containing adenosine deaminase-recruiting RNA (arRNA) used for editing target RNA or a construct for encoding the arRNA is delivered to the target cell, wherein the arRNA comprises a complementary RNA sequence that hybridizes with the target RNA, and can recruit adenosine deaminase acting on RNA (ADAR) to deaminize target adenosine (A) in the target RNA. In some embodiments, the target RNA is precursor mRNA. In some embodiments, the target RNA is mature mRNA. In some embodiments, the target RNA is transcribed RNA comprising the NM_000203.4(IDUA)-c.1205G-A (p.Trp402Ter) mutation site in the IDUA gene.

In some embodiments, the arRNA comprises base C, A, U or G that is paired with the target A. The bases paired with the target A are sorted by priority as C > A > U > G. That is, under the conditions of the same length of arRNA, the same distance between targeting bases and the 5'-terminal, the same distance between the targeting bases and the 3'-terminal, and the completely same arRNA sequence except the targeting bases, the targeting bases are sorted by priority as C > A > U > G. If the targeting base is C, the arRNA can be expressed as X nt-c-Y nt, where, X refers to that a distance between the targeting base and the 5'-terminal is X nt, Y refers to that a distance between the targeting base and the 3'-terminal is Y nt, and X and Y can be any natural number.

In some embodiments, the target cell is a eukaryotic cell. In some embodiments, the target cell is a mammalian cell. In some embodiments, the target cell is hepatocyte or fibroblast. In some embodiments, the target cell is a human or mouse cell.

In some embodiments, the length of the arRNA is about 151-61 nt, 131-66 nt, 121-66 nt, 111-66 nt, 91-66 nt or 81-66 nt. In some embodiments, a distance between a targeting base in the arRNA and the 3'-terminal is 45-5 nt, 40-5 nt, 35-10 nt, 25-15 nt or 24-11 nt. In some embodiments, a distance between the targeting base in the arRNA and the 5'-terminal is 80-30 nt, 70-35 nt, 60-40 nt, 55-35 nt or 55-45 nt. The distance between the targeting base and the 3'-terminal refers to the number of bases from the 3' nearest base of the targeting base to the last base at the 3'-terminal; and the distance between the targeting base and the 5'-terminal refers to the number of bases from the 5' nearest base of the targeting base to the last base at the 5'-terminal.

In some embodiments, if the target cell is a human cell, and the target RNA is transcribed RNA comprising the NM_000203.4(IDUA)-c.1205G-A (p.Trp402Ter) mutation site in the IDUA gene, the full length of the arRNA is equal to or greater than 66 nt, such as about 121-66 nt, 111-66 nt, 101-66 nt, 91-66 nt, and 81-66 nt, that is, the full length of the arRNA is any natural number selected from the above length ranges, such as 67 nt, 68 nt, 69 nt, 70 nt, 71 nt, 72 nt, 73 nt, 74 nt, 75 nt, 76 nt, 77 nt, 78 nt, 79 nt, 80 nt, 81 nt, 82 nt, 83 nt, 84 nt, 85 nt, 86 nt, 87 nt, 88 nt, 89 nt, 90 nt, 91 nt, 95 nt, 100 nt, 110 nt, 115 nt, and 120 nt. In some embodiments, a distance between a targeting base in the arRNA and the 3'-terminal is 45-5 nt, 40-5 nt, 35-10 nt, 25-15 nt or 24-11 nt, that is, the distance between the targeting base in the arRNA and the 3'-terminal is any natural number selected from the above distance ranges, such as 12 nt, 13 nt, 14 nt, 16 nt, 17 nt, 18 nt, 19 nt, 20 nt, 21 nt, 22 nt, and 23 nt. In some embodiments, a distance between the targeting base in the arRNA and the 5'-terminal is 80-30 nt, 70-35 nt, 60-40 nt, 55-35 nt or 55-45 nt, that is, the distance between the targeting base in the arRNA and the 5'-terminal is any natural number selected from the above distance ranges, such as 46 nt, 47 nt, 48 nt, 49 nt, 50 nt, 51 nt, 52 nt, 53 nt, and 54 nt. In some embodiments, the arRNA comprises any one of the following sequences: SEQ ID NO: 14, SEQ ID NO: 15, SEQ ID NO: 9, SEQ ID NO: 13, SEQ ID NO: 17, SEQ ID NO: 22, SEQ ID NO: 23, SEQ ID NO: 30, SEQ ID NO: 31, and SEQ ID NO: 34.

In some embodiments, the target cell is a mouse cell (e.g. W392X mouse cell), and the target RNA is transcribed RNA comprising a mutation, corresponding to the human W402X mutation, in the IDUA gene. In some embodiments, the target cell is a W392X mouse cell. In some embodiments, the length of the arRNA is about 121-53 nt, 111-61 nt, 101-61 nt, 91-61 nt, 81-61 nt, 111-66 nt or 105-66 nt, that is, the full length of the arRNA is any natural number selected from the above length ranges, such as 67 nt, 68 nt, 69 nt, 70 nt, 71 nt, 72 nt, 73 nt, 74 nt, 75 nt, 76 nt, 77 nt, 78 nt, 79 nt, 80 nt, 81 nt, 82 nt, 83 nt, 84 nt, 85 nt, 86 nt, 87 nt, 88 nt, 89 nt, 90 nt, 91 nt, 95 nt, 100 nt, 110 nt, 115 nt, and 120 nt. In some embodiments, a distance between a targeting base in the arRNA and the 3'-terminal is 55-10 nt or 50-10 nt, that is, the distance between the targeting base in the arRNA and the 3'-terminal is any natural number selected from the above length ranges, such as 11 nt, 12 nt, 13 nt, 14 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 35 nt, 37 nt, 38 nt, 39 nt, 40 nt, 41 nt, 42 nt, 43 nt, 44 nt, 45 nt, 46 nt, 47 nt, 48 nt, 49 nt, and 50 nt. In some embodiments, a distance between the targeting base in the arRNA and the 5'-terminal is 80-30 nt, 70-35 nt, 60-40 nt, 55-35 nt or 55-45 nt, that is, the distance between the targeting base in the arRNA and the 5'-terminal is any natural number selected from the above distance ranges, such as 33 nt, 36 nt, 47 nt, 46 nt, 47 nt, 48 nt, 49 nt, 50 nt, 51 nt, 52 nt, 53 nt, 54 nt, 60 nt, 65 nt, and 75 nt. In some embodiments, the arRNA comprises any one of the following sequences: SEQ ID NO: 44 and SEQ ID NO: 52.

In some embodiments, the arRNA is chemically modified. In some embodiments, the chemical modification comprises 2-O'-methylation and/or thiophosphate modification. In some embodiments, the chemical modification is selected from any one or more of:
the first three nucleotides and the last three nucleotides in the sequence are respectively subjected to 2 -OMe modification;
the first three nucleotides and the last three nucleotides are respectively linked via thiophosphate bonds
all bases U in the sequence are subjected to 2'-OMe modification.
the 3' nearest base of the targeting base is A subjected to 2'-OMe modification,
the 5' nearest base of the targeting base is C subjected to 2'-OMe modification,
the targeting base is linked to the 3' nearest base and the 5' nearest base of the targeting base respectively via thiophosphate bonds,
the first five nucleotides and the last five nucleotides are respectively subjected to 2'-OMe modification, and
the first five nucleotides and the last five nucleotides are respectively linked via thiophosphate bonds.

As described herein, the construct for encoding the arRNA is a construct comprising a coding sequence of the arRNA. In some embodiments, the arRNA is generated by transcribing the construct for encoding the arRNA after the construct for encoding the arRNA is delivered to a target cell. In some embodiments, the construct for encoding the arRNA is a linear nucleic acid chain, virus vector or plasmid. In some embodiments, the virus vector is an adeno-associated virus (AAV) vector or lentivirus vector. In some embodiments, after being delivered to the target cell, the construct for encoding the arRNA inserts the coding sequence of the arRNA into the genome of the target cell by homologous recombination or non-homologous recombination so as to continuously generate the arRNA by transcription. In some embodiments, after being delivered to the target cell, the construct for encoding the arRNA enables the coding sequence of the arRNA to exist as a part of a free nucleic acid in the target cell, so that the coding sequence of the arRNA can generate the arRNA by transcription in a certain time period.

In some embodiment, the delivery method is electrotransfection, lipofection, or lipid nanoparticle (LNP) delivery or infection. In some embodiments, if the target cell is hepatocyte, the delivery method is LNP delivery. In some embodiments, if the target cell is fibroblast, the delivery method is lipofection. In some embodiments, the delivery concentration of the arRNA is equal to or greater than 2.5-5 nM, and preferably equal to or greater than 10-20 nM, such as equal to or greater than 15 nM. In the present application, the delivery concentration refers to the arRNA content in one volume unit of a delivery system where one volume unit of arRNA and a target cell are located after the arRNA construct is delivered to the target cell. The delivery system comprises the arRNA or its construct, the target cell, and a fluid matrix around the arRNA and the target cell. In some embodiments, the fluid matrix may be a cell culture medium, PBS or other solutions that have the same osmotic pressure as that of the cytoplasm and can maintain stable survival conditions of cells. In some embodiments, the delivery system further comprises a reagent for promoting delivery.

### arRNA

The present application also provides arRNA, which can be used for LEAPER-based targeted editing of target RNA, such as transcribed RNA comprising the NM_000203.4(IDUA)-c.1205G-A (p.Trp402Ter) mutation site, in a target cell, in order to deaminate target A in the target RNA to inosine (I). In the subsequent translation process of the target cell, I will be identified as G, so that the G>A mutation can be recovered to G, and the target RNA can be translated into a correct protein after being edited by the arRNA. In some embodiments, the target RNA is precursor mRNA. In some embodiments, the target RNA is mature mRNA.

In some embodiments, the arRNA has the base (targeting base) C, A, U or G that is paired with the target A, and the bases are sorted by editing efficiency as C > A > U > G. In some embodiments, in addition to the targeting base, other bases in the arRNA can be paired with the target RNA in a complementary manner. In some embodiments, except the targeting base, one or more bases in the arRNA are mispaired with the target RNA.

In some embodiments, the target cell is a eukaryotic cell. In some embodiments, the target cell is a mammalian cell. In some embodiments, the target cell is hepatocyte or fibroblast. In some embodiments, the target cell is a human or mouse cell (e.g. W392X mouse cell).

In some embodiments, the length of the arRNA is about 151-61 nt, 131-66 nt, 121-66 nt, 111-66 nt, 91-66 nt or 81-66 nt. In some embodiments, a distance between a targeting base in the arRNA and the 3'-terminal is 45-5 nt, 40-5 nt, 35-10 nt, 25-15 nt or 24-11 nt. In some embodiments, a distance between the targeting base in the arRNA and the 5'-terminal is 80-30 nt, 70-35 nt, 60-40 nt, 55-35 nt or 55-45 nt. The distance between the targeting base and the 3'-terminal refers to the number of bases from the 3' nearest base of the targeting base to the last base at the 3'-terminal; and the distance between the targeting base and the 5'-terminal refers to the number of bases from the 5' nearest base of the targeting base to the last base at the 5'-terminal.

In some embodiments, if the target cell is a human cell, and the target RNA is transcribed RNA comprising the NM_000203.4(IDUA)-c.1205G-A (p.Trp402Ter) mutation site in the IDUA gene, the full length of the arRNA is equal to or greater than 66 nt, such as about 121-66 nt, 111-66 nt, 101-66 nt, 91-66 nt, and 81-66 nt, that is, the full length of the arRNA is any natural number selected from the above length ranges, such as 67 nt, 68 nt, 69 nt, 70 nt, 71 nt, 72 nt, 73 nt, 74 nt, 75 nt, 76 nt, 77 nt, 78 nt, 79 nt, 80 nt, 81 nt, 82 nt, 83 nt, 84 nt, 85 nt, 86 nt, 87 nt, 88 nt, 89 nt, 90 nt, 91 nt, 95 nt, 100 nt, 110 nt, 115 nt, and 120 nt. In some embodiments, a distance between a targeting base in the arRNA and the 3'-terminal is 45-5 nt, 40-5 nt, 35-10 nt, 25-15 nt or 24-11 nt, that is, the distance between the targeting base in the arRNA and the 3'-terminal is any natural number selected from the above distance ranges, such as 12 nt, 13 nt, 14 nt, 16 nt, 17 nt, 18 nt, 19 nt, 20 nt, 21 nt, 22 nt, and 23 nt. In some embodiments, a distance between the targeting base in the arRNA and the 5'-terminal is 80-30 nt, 70-35 nt, 60-40 nt, 55-35 nt or 55-45 nt, that is, the distance between the targeting base in the arRNA and the 5'-terminal is any natural number selected from the above distance ranges, such as 46 nt, 47 nt, 48 nt, 49 nt, 50 nt, 51 nt, 52 nt, 53 nt, and 54 nt. In some embodiments, the arRNA comprises any one of the following sequences: SEQ ID NO: 14, SEQ ID NO: 15, SEQ ID NO: 9, SEQ ID NO: 13, SEQ ID NO: 17, SEQ ID NO: 22, SEQ ID NO: 23, SEQ ID NO: 30, SEQ ID NO: 31, and SEQ ID NO: 34.

In some embodiments, if the target cell is a mouse cell (e.g. W392X mouse cell), and the target RNA is transcribed RNA comprising a mutation, corresponding to the human W402X mutation, in the IDUA gene, the length of the arRNA is about 121-53 nt, 111-61 nt, 101-61 nt, 91-61 nt, 81-61 nt, 111-66 nt or 105-66 nt, that is, the full length of the arRNA is any natural number selected from the above length ranges, such as 67 nt, 68 nt, 69 nt, 70 nt, 71 nt, 72 nt, 73 nt, 74 nt, 75 nt, 76 nt, 77 nt, 78 nt, 79 nt, 80 nt, 81 nt, 82 nt, 83 nt, 84 nt, 85 nt, 86 nt, 87 nt, 88 nt, 89 nt, 90 nt, 91 nt, 95 nt, 100 nt, 110 nt, 115 nt, and 120 nt. In some embodiments, a distance between a targeting base in the arRNA and the 3'-terminal is 55-10 nt or 50-10 nt, that is, the distance between the targeting base in the arRNA and the 3'-terminal is any natural number selected from the above distance ranges, such as 11 nt, 12 nt, 13 nt, 14 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 35 nt, 37 nt, 38 nt, 39 nt, 40 nt, 41 nt, 42 nt, 43 nt, 44 nt, 45 nt, 46 nt, 47 nt, 48 nt, 49 nt, and 50 nt. In some embodiments, a distance between the targeting base in the arRNA and the 5'-terminal is 80-30 nt, 70-35 nt, 60-40 nt, 55-35 nt or 55-45 nt, that is, the distance between the targeting base in the arRNA and the 5'-terminal is any natural number selected from the above distance ranges, such as 33 nt, 36 nt, 47 nt, 46 nt, 47 nt, 48 nt, 49 nt, 50 nt, 51 nt, 52 nt, 53 nt, 54 nt, 60 nt, 65 nt, and 75 nt. In some embodiments, the arRNA comprises any one of the following sequences: SEQ ID NO: 44 and SEQ ID NO: 52.

In some embodiments, the arRNA is generated by transcribing and expressing the construct for encoding the arRNA. In some embodiments, the arRNA is obtained by transcribing and expressing the construct for encoding the arRNA in vitro and purifying. In some embodiments, the arRNA is obtained by directly expressing the construct for encoding the arRNA in vivo and used for editing. In some embodiments, the construct is a virus vector, plasmid or linear nucleic acid. In some embodiments, the virus is AAV or lentivirus.

In some embodiments, the arRNA is chemically synthesized. In some embodiments, the arRNA is chemically modified. In some embodiments, the chemical modification comprises 2-O'-methylation and/or thiophosphate modification. In some embodiments, the chemical modification is selected from any one or more of:
the first three nucleotides and the last three nucleotides in the sequence are respectively subjected to 2'-OMe modification;
the first three nucleotides and the last three nucleotides are respectively linked via thiophosphate bonds;
all bases U in the sequence are subjected to 2'-OMe modification;
the 3' nearest base of the targeting base is A subjected to 2'-OMe modification;
the 5' nearest base of the targeting base is C subjected to 2'-OMe modification;
the targeting base is linked to the 3' nearest base and the 5' nearest base of the targeting base respectively via thiophosphate bonds,
the first five nucleotides and the last five nucleotides are respectively subjected to 2'-OMe modification; and
the first five nucleotides and the last five nucleotides are respectively linked via thiophosphate bonds.

In some embodiments, the chemical modification is selected from any one or more of:
CM1, which refers to that the first three nucleotides and the last three nucleotides in the sequence are respectively subjected to 2'-OMe modification, the first three nucleotides and the last three nucleotides are respectively linked via thiophosphate bonds, and meanwhile, all bases U in the sequence are subjected to 2'-OMe modification;
CM2, which refers to that the first three nucleotides and the last three nucleotides in the sequence are respectively subjected to 2'-OMe modification, the first three nucleotides and the last three nucleotides are respectively linked via thiophosphate bonds, and meanwhile, the 3' nearest base of the targeting base is A subjected to 2'-OMe modification;
CM3, which refers to that the first three nucleotides and the last three nucleotides in the sequence are respectively subjected to 2'-OMe modification, the first three nucleotides and the last three nucleotides are respectively linked via thiophosphate bonds, and meanwhile, the 5' nearest base of the targeting base is C subjected to 2'-OMe modification;
CM4, which refers to that the first three nucleotides and the last three nucleotides in the sequence are respectively subjected to 2'-OMe modification, the first three nucleotides and the last three nucleotides are respectively linked via thiophosphate bonds, and meanwhile, the targeting base is linked to the 3' nearest base and the 5' nearest base of the targeting base respectively via thiophosphate bonds; and
CM6, which refers to that the first five nucleotides and the last five nucleotides in the sequence are respectively subjected to 2'-OMe modification, and the first five nucleotides and the last five nucleotides are respectively linked via thiophosphate bonds.

### Construct

The present application also provides a construct for encoding the above arRNA. In some embodiments, the construct is a virus vector, plasmid or linear nucleic acid. In some embodiments, the virus vector is an AAV vector or lentivirus expression vector.

The present application further provides a virus, lipid nanoparticle, liposome, exosome or cell, which comprises the construct.

### Preparation and biological product

The present application also provides a composition, preparation or biological product, which comprises any arRNA or construct in the foregoing description, and can be used for editing of transcribed target RNA comprising the NM_000203.4(IDUA)-c.1205G-A (p.Trp402Ter) mutation site in a target cell, in order to recover normal functions of the IDUA gene. In some embodiments, the arRNA or construct is wrapped within a liposome. In some embodiments, the arRNA or construct is prepared into lipid nanoparticles. In some embodiments, the arRNA or construct is introduced into the body of a subject by virus (e.g. adeno-associated virus or lentivirus) delivery.

In some embodiments, the preparation comprising the arRNA is a therapeutic agent, which can be infused into the body of a patient to treat a disease. In some embodiments, the infusion method is local injection, local perfusion or intravenous infusion, or local perfusion or local injection. In some embodiments, the therapeutic agent is in a form applicable to local injection into the liver, such as arterial perfusion into the liver. In some embodiments, the therapeutic agent is in a form applicable to intramuscular injection. In some embodiments, the therapeutic agent is in a form applicable to intravenous injection.

### Kit

The present application also provides a kit used for editing of target RNA in a target cell, which comprises the above arRNA, construct for encoding the arRNA or preparation. The kit can be used for editing of transcribed target RNA comprising the NM_000203.4(IDUA)-c.1205G-A (p.Trp402Ter) mutation site in the target cell.

In some embodiments, the kit comprises the above arRNA or construct for encoding the arRNA, and a dyeing auxiliary, which are packaged in different containers. In some embodiments, the dyeing auxiliary is a lipid solution. For example, the dyeing auxiliary is Lipo (lipofectmine RNAiMAX, catalog No. 13778150) purchased from Invitrogen or a reagent comprising the same functional ingredients.

In some embodiments, the kit further comprises an instruction for introducing various ingredients and the content in the kit to users, and/or the use method of the kit.

### Treatment method

The present application further provides a method for treating mucopolysaccharidosis IH in an individual, which comprises: correcting a G-to-A mutation in the IDUA gene, such as the NM_000203.4(IDUA)-c.1205G-A (p.Trp402Ter) mutation, in the cell of an individual by the above method. In some embodiments, the diseases include Hurler syndrome. In some embodiments, if the individual is a human, the arRNA is administrated at a frequency of ≥every 21 days, ≥every 17 days, ≥every 14 days or ≥every 10 days. In some embodiments, if the individual is a mouse, the arRNA is administrated at a frequency of ≥every 8 days. In some embodiments, the treatment method uses a construct for encoding the arRNA, and the construct can integrate a coding sequence of the arRNA into the target cell, and the arRNA is administrated once.

The LEAPER-based method for targeted editing of RNA provided in the present application has the following advantages.
1. The method does not rely on the expression of exogenous proteins, so it avoids difficulty in loading and in vivo delivery via virus vectors due to the large molecular weight of proteins, avoids an off-target effect caused by the overexpression of exogenous proteins, avoids an immune response and injury of an organism caused by the expression of exogenous proteins, and avoids the failure of gene editing caused by neutralization of exogenous enzymes used for editing or effector proteins with antibodies pre-existing in the organism.
2. The enzyme-guided site-specific RNA editing method provided in the present application is different from DNA editing, RNA editing is reversible and controllable. Diseases can be treated and functions of proteins and RNA can be studied by recoding amino acid codons. Potential side effects of RNA editing are reversible, so RNA editing is safer.
3. Compared with the prior art, the method can chemically synthesize the arRNA, and deliver the arRNA to a patient by electrotransfection or lipofection, or via a vector such as an adeno-associated virus or lentivirus, the delivery methods are flexible and changeable, and the editing efficiency is higher.

The preferred embodiments of the present application have been described above, but the present application is not limited thereto. Many simple modifications including combinations of various technical features in any other suitable manners can be made within the scope of the technical idea of the present application, and these simple modifications and combinations shall be also regarded as the content disclosed in the present application and fall within the scope of protection of the present application.

The technical solutions of the present application will be further described in detail below with reference to specific examples, but the present application is not limited to the following examples. Unless otherwise defined, reagents involved in the following description are commercially available products. For brevity, some experiments are described without specifying parameters, steps and instruments used, and it should be understood that these experiments are well known and reproducible by those skilled in the art. Cells (GM06214) used herein are purchased from Coriell (USA), and are fibroblasts from patients with Hurler syndrome. arRNA used for editing is synthesized by Synthego (USA) or Biosyntech (Suzhou), the Sanger sequencing is performed by RuiBioTech (Beijing), the next generation sequencing is performed by Novogene or the sequencing platform of China National Rice Research Institute.

### EXAMPLES

### Example 1: Detection of the mutation genotype of GM06214 cells

GM06214 cells (fibroblasts from patients with Hurler syndrome) were placed into a fibroblast culture solution comprising 15% serum (ScienCell, FM, catalog No. 2301), a 1% fibroblast growth supplement (ScienCell, FGS, catalog No. 2301) was added, and the cells were cultured in an incubator with 5% CO₂ at 37°C for 2-3 d. After growing to 90% confluence, the cells were digested with 0.25% pancreatic enzyme, and then the digestion was terminated with a fibroblast culture solution comprising 15% serum. DNA was extracted by using a TianGene^{®} (TIANGEN Biotech (Beijing) Co., Ltd.) DNA extraction kit (catalog No. DP304-03) according to the instruction.

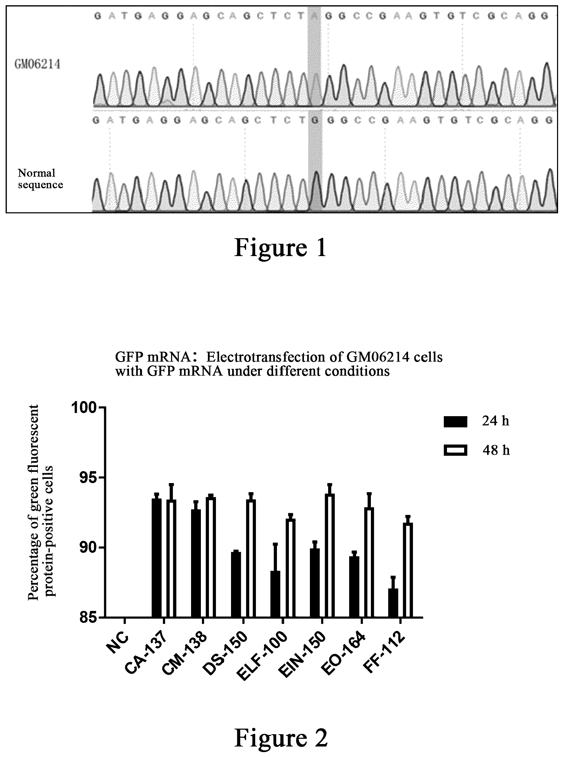

Primers for the upstream and downstream sequences of a mutation site in the IDUA gene were designed on NCBI-Primer blast (https://www.ncbi.nlm.nih.gov/tools/primer-blast/). SEQ ID NO: 1 CGCTTCCAGGTCAACAACAC (forward primer hIDUA-F1); SEQ ID NO: 2 CTCGCGTAGATCAGCACCG (reverse primer hIDUA-R1). PCR reaction is performed, and then the PCR product was subjected to Sanger sequencing. It was determined that the mutation genotype of the cells was a pathogenic G-to-A mutation at the 15704 site in the IDUA genome, as shown in Fig. 1.

### Example 2: Screening of GM06214 cell electrotransfection conditions

After growing to about 90% confluence, GM06214 cells were digested, and after the digestion was terminated, the cells were counted. During electrotransfection, 6 million cells were resuspended with 400 µL of pre-mixed electrotransfection solution (Lonza, catalog No. V4XP-3024), 20 µg of GFP plasmids (Lonza, catalog No. V4XP-3024) was added to and uniformly mixed with the cells, 20 uL of electrotransfection system was taken as a sample and detected on an Lonza nuclear electrotransfection instrument under eight conditions including seven electrotransfection conditions (see Fig. 2) and one negative control condition, and double determination were performed under each condition. After electrotransfection, the cells were immediately transferred to 2 mL of fibroblast culture solution comprising 15% serum (ScienCell, FM, catalog No. 2301), the cells of each condition were inoculated into 2 wells of a 6-well plate, and the cells were cultured in the incubator with 5% CO₂ at 37°C. 24 h after electrotransfection, cells from one well from the 2 wells of each electrotransfection condition were digested, and the proportion of GFP-positive cells was detected by using a flow cytometer. 48 h after electrotransfection, cells from another well from the 2 wells of each electrotransfection condition were digested, and the proportion of GFP-positive cells was detected by using the flow cytometer. The optimal cell electrotransfection condition obtained was the electrotransfection condition of the CA-137 group, as shown in Fig. 2.

### Example 3: IDUA enzymatic activity and editing efficiency in GM06214 cells after electrotransfection with arRNA

The following arRNA sequences were designed and synthesized for the upstream and downstream sequences of mutation sites on pre-mRNA (precursor mRNA) and mature-mRNA (mature mRNA) after transcription of the IDUA gene: SEQ ID NO: 3 GACGCCCACCGUGUGG
UUGCUGUCCAGGACGGUCCCGGCCUGCGACACUUCGGCCCAGAGCUGCUCCUC AUCCAGCAGCGCCAGCAGCCCCAUGGCCGUGAGCACCGGCUU (Pre-55nt-c-55nt);
SEQ ID NO: 4 GACGCCCACCGUGUGGUUGCUGUCCAGGACGGUCCCGGCCUGCGACACUUCGG CCC
AGAGCUGCUCCUCAUCUGCGGGGCGGGGGGGGGCCGUCGCCGCGUGGGGUCGU UG (m-55nt-c-55nt); and SEQ ID NO: 5 UACCGCUACAGCCACGCUGAUUUCAGCUAUACCUGC
CCGGUAUAAAGGGACGUUCACACCGCGAUGUUCUCUGCUGGGGAAUUGCGCGA UAUUCAGGAUUAAAAGAAGUGC (Random-111nt). A base, corresponding to the mutation site, in the synthesized arRNA was converted from T to C so as to form A-C mispairing. The length of the synthesized arRNA was preferably 111 nt. Cells were subjected to electrotransfection under the optimal electrotransfection condition obtained in Example 2, and 48 h after electrotransfection, the cells were collected and subjected to enzymatic activity assay and editing efficiency detection.

### Detection of the editing efficiency:

The designed and synthesized arRNA was dissolved to the required concentration in RNase-free water (TransGen, catalog No. GI201-01) and stored at -80°C. After growing to about 90% confluence, GM06214 cells were digested, and after digestion was terminated, the cells were counted. 1 million cells were added with 200 pmol of arRNA until the volume of the mixture was 100 uL, the cells were subjected to electrotransfection under the CA-137 condition. 48 h after the electrotransfection, the cells were counted and subjected to cell viability assay. The cells were transferred into an RNA enzyme-free centrifuge tube and centrifuged, a supernate was removed, and RNA was extracted by using a QIAGEN RNA extraction kit (QIAGEN, catalog No. 74134). According to an operation manual, 5×10⁵ cells were added to and uniformly mixed with 0.35 mL of Buffer RLT Plus by being pipetted evenly (for cryopreserved cells, RNA is directly extracted, and it is recommended to wash once with PBS). The lysed cell solution was added to a gDNA Eliminator spin column and centrifuged at ≥ 8000 g for 30 s, the column was removed, and a fluid was collected. 70% ethanol with the same volume as that of the fluid was added to and uniformly mixed with the fluid by being pipetted, and the next step was performed. The liquid was added to an RNeasyMinElute spin column and centrifuged at ≥ 8000 g for 15 s, and liquid waste was removed. 700 µL of Buffer RW1 was added to the RNeasyMinElute spin column and centrifuged at ≥ 8000 g for 15 s, and liquid waste was removed. 500 µL of Buffer RPE was added to the RNeasyMinElute spin column and centrifuged at ≥ 8000 g for 15 s, and liquid waste was removed. 500 µL of 80% ethanol was added to the RNeasyMinElute spin column and centrifuged at ≥ 8000 g for 2 min, and liquid waste was removed. The RNeasyMinElute spin column was placed into a new 2 mL collection column and centrifuged without being covered at the highest relative centrifugal force for 5 min to be dried. The RNeasyMinElute spin column was placed into a new 1.5 mL collection column, 14 µL of RNase-free water was dropwise added to the center of the column membrane, and the column was centrifuged at the highest speed for 1 min to dilute RNA.

The concentration of the extracted RNA was detected by using Nanodrop (Thermo, catalog No. Nanodrop2000), and 1 µg of RNA was subjected to reverse transcription (Thermo, reverse transcriptase catalog No. 28025013). A reverse transcription system was prepared according to Table 1, incubated at 65°C for 5 min, and immediately cooled in an ice bath. The reverse transcription system was incubated again at 37°C for 50 min. The reverse transcriptase was inactivated at 70°C for 15 min. PCR was performed under the conditions shown in Table 3. After the PCR was completed, 2 µL of PCR product was subjected to agarose gel electrophoresis, and the correctness of the concentration of the PCR product and the size of the band was preliminarily determined according to electrophoresis results. After being purified, the library was established with the PCR products and these products were subjected to the next generation sequencing.

**Table 1 Preparation of a reverse transcription system-1**

| | Volume (µL) |
|---|---|
| Total RNA (1 µg) | X |
| Oligo dT | 1 |
| 10 nM dNTP | 1 |
| RNase-Free Water | 10-X |
| Total volume | 12 |

The reaction system run at 65°C for 5 min, and then was immediately transferred onto ices.

**Table 2 Preparation of a reverse transcription system-2**

| | Volume (µL) |
|---|---|
| The system in Table 1 | 12 µL |
| 5X First-Strand Buffer | 4 |
| 0.1 MDTT | 2 |
| RNaseOUT^{™} Recombinant Ribonuclease Inhibitor | 1 |
| M-MLV | 1 |
| Total volume | 20 |

**Table 3 PCR conditions**

| Steps | Time | Cycles |
|---|---|---|
| 98°C | 2 min | 1 cycle |
| 98°C | 15 s | 28-35 cycles |
| 63°C | 30 s | |
| 72°C | 15 s | |
| 72°C | 2 min | 1 cycle |

### Enzymatic activity assay

GM06214 cells were digested, centrifuged, resuspended with 28 µL of 1×PBS comprising 0.1% Triton X-100, and lysed on ices for 30 min. 25 µL of lysed cell solution was added to 25 µL of substrate (Cayman, 2A-19543-500, dissolved in a 0.4 M sodium formate solution, comprising 0.2% Triton X-100, pH=3.5) comprising 190 µm of 4-methylumbelliferyl-α-L-iduronidase. The cells were incubated in the dark at 37°C for 90 min. 200 µL of 0.5M NaOH/Glycine solution (Beijing Chemical Technology, NAOH, catalog No. AR500G; Solarbio, Glycine, catalog No. G8200) (pH=10.3) was added to inactivate the catalysis reaction. The cells were centrifuged at 4°C for 2 min. A supernate was transferred into a 96-well plate and subjected to fluorescence measurement at the excitation wavelengths of 365 nm and 450 nm by using an Infinite M200 instrument (TECAN).

In the present application, all result data of the enzymatic activity assay are expressed as folds of the enzyme activity in GM01323 cells. The GM01323 cells are fibroblasts from patients with Scheie syndrome. Scheie syndrome is the mildest subtype of mucopolysaccharidosis and is much milder than Hurler syndrome. Patients with Scheie syndrome usually have better prognosis and a normal lifespan, and can survive to adulthood. The IDUA enzyme activity in fibroblasts of patients with Scheie syndrome is 0.3% of that in wild-type fibroblasts of healthy people.

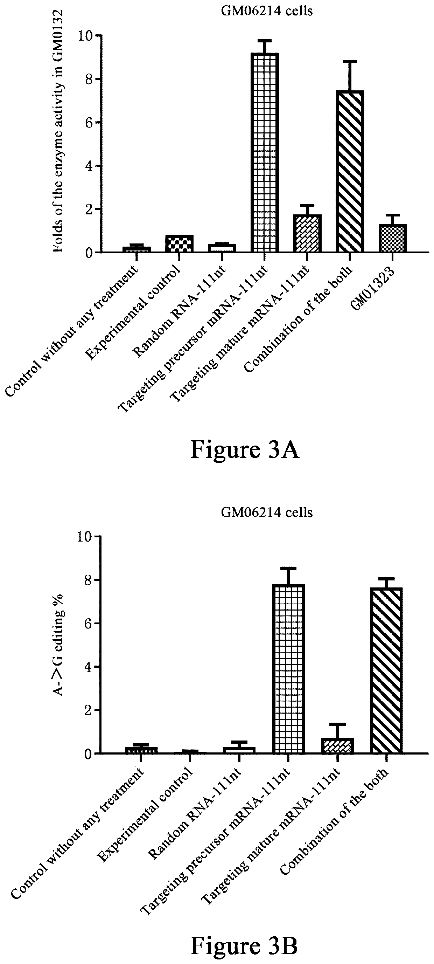

As shown in Fig. 3, the arRNA targeting precursor mRNA (pre-mRNA) can cause high enzymatic activity and editing efficiency, and the arRNA targeting mature mRNA (mature-mRNA) causes significantly low enzymatic activity and editing efficiency. Therefore, arRNAs involved in the following examples are arRNAs targeting precursor mRNA (pre-mRNA).

### Example 4: Detection of the editing efficiency at an IDUA target site after electrotransfection of an IDUA-reporter cell line with arRNA

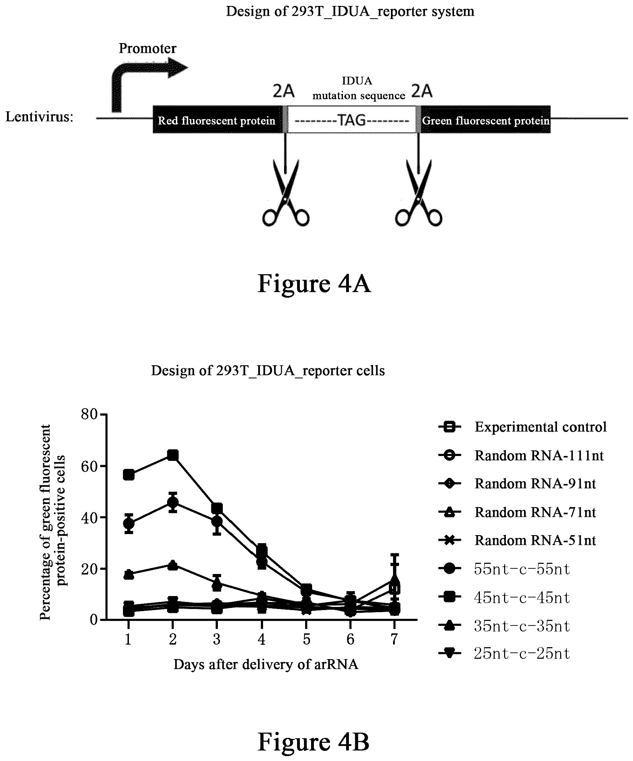

As shown in Fig. 4A, a plasmid was constructed by inserting a sequence carrying an IDUA mutation site and having about 100 bp upstream and 100 bp downstream between sequences expressing mCherry and GFP proteins on a lentivirus plasmid. The above constructed plasmid was prepared into a virus and used to infect 293T cells, and after the sequence was integrated into the genome, IDUA-reporter monoclonal cells were screened out. The monoclonal cells can only express the mCherry protein because of the influence of a termination codon TAG of the IDUA mutation site in the inserted sequence, after the cells are edited by arRNA, TAG->TGG occurs, the GFP protein located behind the mutation site can be normally expressed, and the expression of the GFP protein can be regarded as the editing efficiency of the arRNA in the cells. 4 arRNAs with different lengths of 51-111 nt were preferably designed, as shown in Table 4, after electrotransfection of the cells with the arRNAs of different lengths under the electrotransfection condition of Example 2, the proportion of GFP in the cells were detected from day 1 to day 7, and the editing efficiency was preliminarily detected. As shown in Fig. 4B, the editing efficiency of the sequence 91nt: 45nt-c-45nt is the highest, and the peak of editing appears on day 2 (48 h). It indicates that in terms of the length of arRNA, it is not the longer the sequence the higher the editing efficiency.

**Table 4**

| | |
|---|---|
| 111nt-random | SEQ ID NO: 5 uaccgcuacagccacgcugauuucagcuauaccugcccgguauaaagggacguucacaccgcgauguu cucugcuggggaauugcgcgauauucaggauuaaaagaagugc |
| 91nt-random | SEQ ID NO: 6 uaauccugaauaucgcgcaauuccccagcagagaacaucgcggugugaacgucccuuuauaccgggca gguauagcugaaaucagcguggc |
| 71nt-random | SEQ ID NO: 7 uuucagcuauaccugcccgguauaaagggacguucacaccgcgauguucucugcuggggaauugcgc gaua |
| 51nt-random | SEQ ID NO: 8 uuccccagcagagaacaucgcggugugaacgucccuuuauaccgggcaggu |
| 55nt-c-55nt | SEQ ID NO: 4 gacgcccaccgugugguugcuguccaggacggucccggccugcgacacuucggcccagagcugcucc ucaucugcggggcgggggggggccgucgccgcguggggucguug |
| 45nt-c-45nt | SEQ ID NO: 9 guguggguugcuguccaggacggucccggccugcgacacuucggcccagagcugcuccucaucugcgg ggcgggggggggccgucgccgcgu |
| 35nt-c-35nt | SEQ ID NO: 10 uguccaggacggucccggccugcgacacuucggcccagagcugcuccucaucugcggggcggggggg ggcc |
| 25nt-c-25nt | SEQ ID NO: 11 ggucccggccugcgacacuucggcccagagcugcuccucaucugcggggcg |

### Example 5: IDUA enzymatic activity assay and Detection of the RNA editing efficiency in GM06214 cells at different time points after electrotransfection with arRNAs of different lengths

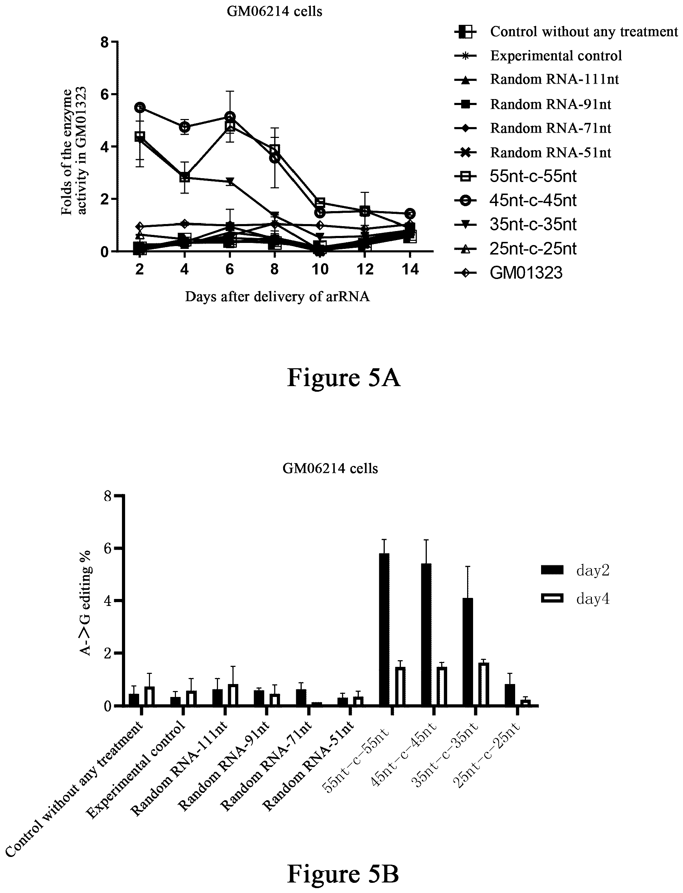

Electrotransfection of GM06214 cells with arRNAs of different lengths (see Table 4) was performed under the electrotransfection condition of Example 2, the enzymatic activity in the cells was assayed on day 2, day 4, day 6, day 8, day 10, day 12, and day 14 respectively after electrotransfection by the method of Example 3, and the RNA editing efficiency in the cells was detected on day 2 and day 4. As shown in Fig. 5, the enzymatic activity caused by the sequence 91 nt: 45nt-c-45nt is the highest, and the IDUA enzymatic activity still maintains at a high level on day 6 after electrotransfection. With regard to the editing efficiency, the sequences of 91 nt and 111 nt exhibit approximately the same editing efficiency.

### Example 6: Detection of the editing efficiency of a targeting base at different sites on arRNA arRNA with the length of 111 nt was taken as an example, the editing efficiency of a targeting base at different site on arRNA was detected by cutting off from both sides of a mutation site at the same time and cutting out from the 5'-terminal or the 3'-terminal.

In the present example, the arRNA was introduced into cells by lipofectmine RNAiMAX. First, the arRNA sequence was truncated from both terminals at the same time, and then one terminal was fixed and the other terminal was truncated to obtain 14 arRNAs and 4 random sequences of the same length, as shown in Table 5. 48 h after transfection, the IDUA enzymatic activity was assayed and the RNA editing efficiency was detected. As shown in Fig. 6, the IDUA enzymatic activity and the RNA editing efficiency caused by the sequences 81 nt: 55nt-c-25nt (SEQ ID NO: 14), 71 nt: 55nt-c-15nt (SEQ ID NO: 15), 91 nt: 45nt-c-45nt (SEQ ID NO: 9), 91 nt: 55nt-c-35nt (SEQ ID NO: 13), and 101 nt: 45nt-c-55nt (SEQ ID NO: 17) are higher than those caused by other sequences.

**Table 5**

| | |
|---|---|
| 111nt-random | SEQ ID NO: 5 uaccgcuacagccacgcugauuucagcuauaccugcccgguauaaagggacguucacaccgcgaugu ucucugcuggggaauugcgcgauauucaggauuaaaagaagugc |
| 91nt-random | SEQ ID NO: 6 uaauccugaauaucgcgcaauuccccagcagagaacaucgcggugugaacgucccuuuauaccgggc |
| | agguauagcugaaaucagcguggc |
| 71nt-random | SEQ ID NO: 7 uuucagcuauaccugcccgguauaaagggacguucacaccgcgauguucucugcuggggaauugcg cgaua |
| 51nt-random | SEQ ID NO: 8 uuccccagcagagaacaucgcggugugaacgucccuuuauaccgggcaggu |
| 55nt-c-55nt | SEQ ID NO: 4 gacgcccaccgugugguugcuguccaggacggucccggccugcgacacuucggcccagagcugcuc cucaucugcggggcgggggggggccgucgccgcgugggggucguug |
| 45nt-c-45nt | SEQ ID NO: 9 guguggguugcuguccaggacggucccggccugcgacacuucggcccagagcugcuccucaucugcg gggcgggggggggccgucgccgcgu |
| 35nt-c-35nt | SEQ ID NO: 10 uguccaggacggucccggccugcgacacuucggcccagagcugcuccucaucugcggggcgggggg gggcc |
| 25nt-c-25nt | SEQ ID NO: 11 ggucccggccugcgacacuucggcccagagcugcuccucaucugcggggcg |
| 55nt-c-45nt | SEQ ID NO: 12 gacgcccaccgugugguugcuguccaggacggucccggccugcgacacuucggcccagagcugcuc cucaucugcggggcgggggggggccgucgccgcgu |
| 55nt-c-35nt | SEQ ID NO: 13 gacgcccaccguguggguugcuguccaggacggucccggccugcgacacuucggcccagagcugcuc cucaucugcggggcgggggggggcc |
| 55nt-c-25nt | SEQ ID NO: 14 gacgcccaccgugugguugcuguccaggacggucccggccugcgacacuucggcccagagcugcuc cucaucugcggggcg |
| 55nt-c-15nt | SEQ ID NO: 15 gacgcccaccgugugguugcuguccaggacggucccggccugcgacacuucggcccagagcugcuc cucau |
| 55nt-c-5nt | SEQ ID NO: 16 gacgcccaccgugugguugcuguccaggacggucccggccugcgacacuucggcccagagc |
| 45nt-c-55nt | SEQ ID NO: 17 guguggguugcuguccaggacggucccggccugcgacacuucggcccagagcugcuccucaucugcg gggcgggggggggccgucgccgcgugggggucguug |
| 35nt-c-55nt | SEQ ID NO: 18 uguccaggacggucccggccugcgacacuucggcccagagcugcuccucaucugcggggcgggggg gggccgucgccgcguggggucguug |
| 25nt-c-55nt | SEQ ID NO: 19 ggucccggccugcgacacuucggcccagagcugcuccucaucugcggggcgggggggggccgucgc cgcguggggucguug |
| 15nt-c-55nt | SEQ ID NO: 20 ugcgacacuucggcccagagcugcuccucaucugcggggcgggggggggccgucgccgcgugggg ucguug |
| 5nt-c-55nt | SEQ ID NO: 21 cggcccagagcugcuccucaucugcggggcgggggggggccgucgccgcguggggucguug |

### Example 7: Influence of a distance between a targeting base and the 3'-terminal on the editing efficiency

By Example 6, it was found that the IDUA enzymatic activity and the editing efficiency caused by the sequences 81 nt: 55nt-c-25nt and 71 nt: 55nt-c-15nt were higher. In order to screen out the shortest and optimal distance between a targeting base and the 3'-terminal, the distance between the targeting base and the 3'-terminal was gradually truncated from 25 nt (81 nt: 55nt-c-25nt) to 10 nt (66 nt: 55nt-c-10nt), as shown in Table 6. As shown in Fig. 7A, by IDUA enzymatic activity assay, it is found that the sequence 24nt-c-11nt has the optimal distance between the targeting base and the 3'-terminal. In addition, by comparison, it can be seen that the enzyme activity caused by the sequences 80nt: 55nt-c-24nt (SEQ ID NO: 22), 79 nt: 55nt-c-23nt (SEQ ID NO: 23), 72 nt: 55nt-c-16nt (SEQ ID NO: 30), 70 nt: 55nt-c-14nt (SEQ ID NO: 31), and 67 nt: 55nt-c-11nt (SEQ ID NO: 34) is similar to or higher than that caused by SEQ ID NO: 14 and SEQ ID NO: 15.

Moreover, the screening of the optimal distance between a targeting base and the 3'-terminal was performed on arRNA targeting a mouse IDUA mutation site (corresponding to the human IDUA-W402X mutation in a human), the distance between the targeting base in the arRNA and the 3'-terminal was truncated by every 5 bases from 55 nt, as shown in Table 7. As shown in Fig. 7B, by IDUA enzyme activity assay, it is found that the sequence 55nt-c-10nt has the optimal distance between the targeting base and the 3'-terminal. In addition, the sequences 111 nt: 55nt-c-50nt (SEQ ID NO: 44) and 66nt: 55nt-c-10nt (SEQ ID NO: 52) exhibit high editing efficiency.

**Table 6**

| | |
|---|---|
| 55nt-c-25nt | SEQ ID NO: 14 gacgcccaccgugugguugcuguccaggacggucccggccugcgacacuucggcccaga gcugcuccucaucugcggggcg |
| 55nt-c-24nt | SEQ ID NO: 22 gacgcccaccgugugguugcuguccaggacggucccggccugcgacacuucggcccaga gcugcuccucaucugcggggc |
| 55nt-c-23nt | SEQ ID NO: 23 gacgcccaccgugugguugcuguccaggacggucccggccugcgacacuucggcccaga gcugcuccucaucugcgggg |
| 55nt-c-22nt | SEQ ID NO: 24 gacgcccaccgugugguugcuguccaggacggucccggccugcgacacuucggcccaga gcugcuccucaucugcggg |
| 55nt-c-21nt | SEQ ID NO: 25 gacgcccaccguguggguugcuguccaggacggucccggccugcgacacuucggcccaga gcugcuccucaucugcgg |
| 55nt-c-20nt | SEQ ID NO: 26 gacgcccaccgugugguugcuguccaggacggucccggccugcgacacuucggcccaga gcugcuccucaucugcg |
| 55nt-c-19nt | SEQ ID NO: 27 gacgcccaccguguggguugcuguccaggacggucccggccugcgacacuucggcccaga |
| | gcugcuccucaucugc |
| 55nt-c-18nt | SEQ ID NO: 28 gacgcccaccgugugguugcuguccaggacggucccggccugcgacacuucggcccaga gcugcuccucaucug |
| 55nt-c-17nt | SEQ ID NO: 29 gacgcccaccgugugguugcuguccaggacggucccggccugcgacacuucggcccaga gcugcuccucaucu |
| 55nt-c-16nt | SEQ ID NO: 30 gacgcccaccgugugguugcuguccaggacggucccggccugcgacacuucggcccaga gcugcuccucauc |
| 55nt-c-15nt | SEQ ID NO: 15 gacgcccaccgugugguugcuguccaggacggucccggccugcgacacuucggcccaga gcugcuccucau |
| 55nt-c-14nt | SEQ ID NO: 31 gacgcccaccgugugguugcuguccaggacggucccggccugcgacacuucggcccaga gcugcuccuca |
| 55nt-c-13nt | SEQ ID NO: 32 gacgcccaccgugugguugcuguccaggacggucccggccugcgacacuucggcccaga gcugcuccuc |
| 55nt-c-12nt | SEQ ID NO: 33 gacgcccaccgugugguugcuguccaggacggucccggccugcgacacuucggcccaga gcugcuccu |
| 55nt-c-11nt | SEQ ID NO: 34 gacgcccaccgugugguugcuguccaggacggucccggccugcgacacuucggcccaga gcugcucc |
| 55nt-c-10nt | SEQ ID NO: 35 gacgcccaccgugugguugcuguccaggacggucccggccugcgacacuucggcccaga gcugcuc |

**Table 7**

| | | |
|---|---|---|
| Mous | 55nt-c-55nt | GacacccacUgUaUgaUUgcUgUccaacacagccccagccUUUgagaccUc |
| e arRN A | | UgcccagagUUgUUcUccaUcUaUaagccaagcagagggcUgaggcUgU UggcUcUcUca (SEQ ID NO: 62) |
| | 55nt-c-50nt | GacacccacUgUaUgaUUgcUgUccaacacagccccagccUUUgagaccUc UgcccagagUUgUUcUccaUcUaUaagccaagcagagggcUgaggcUgU UggcUc (SEQ ID NO: 44) |
| | 55nt-c-45nt | GacacccacUgUaUgaUUgcUgUccaacacagccccagccUUUgagaccUc UgcccagagUUgUUcUccaUcUaUaagccaagcagagggcUgaggcUgU U (SEQ ID NO: 45) |
| | 55nt-c-40nt | GacacccacUgUaUgaUUgcUgUccaacacagccccagccUUUgagaccUc UgcccagagUUgUUcUccaUcUaUaagccaagcagagggcUgagg (SEQ ID NO: 46) |
| | 55nt-c-35nt | gacacccacUgUaUgaUUgcUgUccaacacagccccagccUUUgagaccUc UgcccagagUUgUUcUccaUcUaUaagccaagcagagggc (SEQ ID NO: 47) |
| | 55nt-c-30nt | gacacccacUgUaUgaUUgcUgUccaacacagccccagccUUUgagaccUc UgcccagagUUgUUcUccaUcUaUaagccaagcag (SEQ ID NO: 48) |
| | 55nt-c-25nt | gacacccacUgUaUgaUUgcUgUccaacacagccccagccUUUgagaccUc UgcccagagUUgUUcUccaUcUaUaagcca (SEQ ID NO: 49) |
| | 55nt-c-20nt | gacacccacUgUaUgaUUgcUgUccaacacagccccagccUUUgagaccUc UgcccagagUUgUUcUccaUcUaUa (SEQ ID NO: 50) |
| | 55nt-c-15nt | gacacccacUgUaUgaUUgcUgUccaacacagccccagccUUUgagaccUc UgcccagagUUgUUcUccaU (SEQ ID NO: 51) |
| | 55nt-c-10nt | gacacccacUgUaUgaUUgcUgUccaacacagccccagccUUUgagaccUc UgcccagagUUgUUc (SEQ ID NO: 52) |

### Example 8: Influence of the length of the 5'-terminal on the editing efficiency

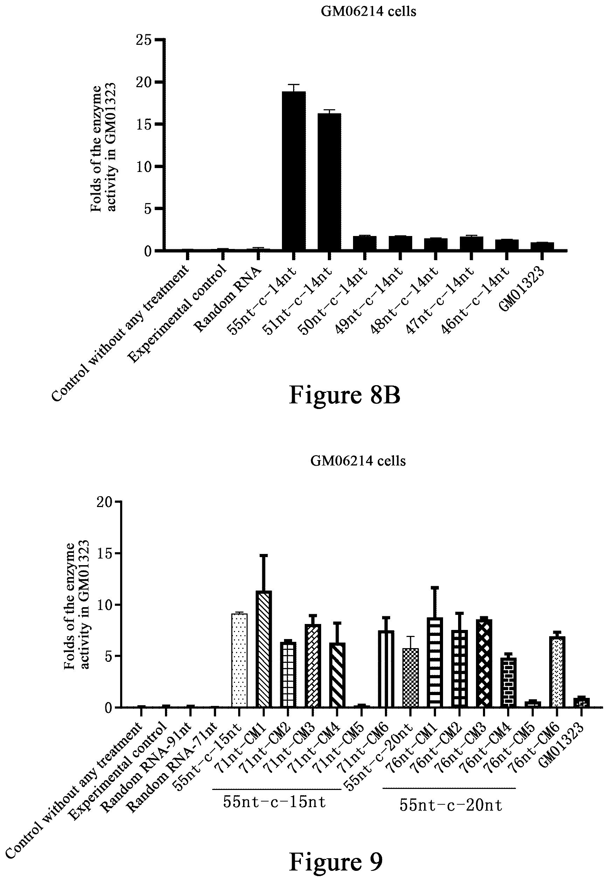

2 arRNAs of different lengths, i.e. 76 nt: 55nt-c-20nt and 71 nt: 55nt-c-15nt, were selected, the length of the 3'-terminal is kept unchanged, the length of the 5'-terminal was gradually truncated, as shown in Table 8. As shown in Fig. 8A, by IDUA enzymatic activity assay, it is found that if the length of the 5'-terminal is 55-45 nt, the IDUA enzymatic activity in cells edited by the arRNA is high, and if the full length of the arRNA is 65-61 nt, the IDUA enzymatic activity decreases significantly. The length of the 3'-terminal is fixed at 14 nt, the 5'-terminal is gradually truncated base by base from 51 nt (the full length was 66 nt), and when the 5'-terminal was truncated from 51 nt to 50 nt (the full length was 65 nt), the IDUA enzymatic activity decreased significantly. Therefore, truncation of the 5'-terminal requires that the full length of arRNA is not less than 66 nt, as shown in Fig. 8B.

**Table 8**

| | |
|---|---|
| 55nt-c-20nt | SEQ ID NO: 26 gacgcccaccgugugguugcuguccaggacggucccggccugcgacacuucggcccagagcugcucc ucaucugcg |
| 50nt-c-20nt | SEQ ID NO: 36 ccaccguguggguugcuguccaggacggucccggccugcgacacuucggcccagagcugcuccucauc ugcg |
| 45nt-c-20nt | SEQ ID NO: 37 gugugguugcuguccaggacggucccggccugcgacacuucggcccagagcugcuccucaucugcg |
| 40nt-c-20nt | SEQ ID NO: 38 guugcuguccaggacggucccggccugcgacacuucggcccagagcugcuccucaucugcg |
| 35nt-c-20nt | SEQ ID NO: 39 uguccaggacggucccggccugcgacacuucggcccagagcugcuccucaucugcg |
| 55nt-c-15nt | SEQ ID NO: 15 gacgcccaccgugugguugcuguccaggacggucccggccugcgacacuucggcccagagcugcucc ucau |
| 50nt-c-15nt | SEQ ID NO: 40 ccaccgugugguugcuguccaggacggucccggccugcgacacuucggcccagagcugcuccucau |
| 45nt-c-15nt | SEQ ID NO: 41 gugugguugcuguccaggacggucccggccugcgacacuucggcccagagcugcuccucau |
| 40nt-c-15nt | SEQ ID NO: 42 guugcuguccaggacggucccggccugcgacacuucggcccagagcugcuccucau |
| 35nt-c-15nt | SEQ ID NO: 43 uguccaggacggucccggccugcgacacuucggcccagagcugcuccucau |
| 51nt-c-14nt | SEQ ID NO: 56 cccaccgugugguugcuguccaggacggucccggccugcgacacuucggcccagagcugcuccuca |
| 50nt-c-14nt | SEQ ID NO: 57 ccaccgugugguugcuguccaggacggucccggccugcgacacuucggcccagagcugcuccuca |
| 49nt-c-14nt | SEQ ID NO: 58 caccgugugguugcuguccaggacggucccggccugcgacacuucggcccagagcugcuccuca |
| 48nt-c-14nt | SEQ ID NO: 59 accgugugguugcuguccaggacggucccggccugcgacacuucggcccagagcugcuccuca |
| 47nt-c-14nt | SEQ ID NO: 60 ccgugugguugcuguccaggacggucccggccugcgacacuucggcccagagcugcuccuca |
| 46nt-c-14nt | SEQ ID NO: 61 cgugugguugcuguccaggacggucccggccugcgacacuucggcccagagcugcuccuca |

### Example 9: Influence of chemical modifications to RNA on the editing efficiency

Different chemical modifications to RNA during RNA synthesis can increase the stability of the RNA and reduce the possibility of off-target. Common chemical modifications to RNA include 2'-OMe and thiophosphate modification. In the present example, 2 arRNAs of different lengths, i.e. 71 nt and 76 nt, were selected, and different combinations of the two chemical modification methods were performed, as shown in Table 8. Specific modification methods were as follows.
CM1: the first three nucleotides and the last three nucleotides in the sequence were respectively subjected to 2'-OMe modification, the first three nucleotides and the last three nucleotides were respectively linked via thiophosphate bonds, and meanwhile, all bases U in the sequences were subjected to 2'-OMe modification.
CM2: the first three nucleotides and the last three nucleotides in the sequence were respectively subjected to 2'-OMe modification, the first three nucleotides and the last three nucleotides were respectively linked via thiophosphate bonds, and meanwhile, the 3' nearest base of a targeting base was A subjected to 2'-OMe modification.
CM3: the first three nucleotides and the last three nucleotides in the sequence were respectively subjected to 2'-OMe modification, the first three nucleotides and the last three nucleotides were respectively linked via thiophosphate bonds, and meanwhile, the 5' nearest base of the targeting base was C subjected to 2'-OMe modification.
CM4: the first three nucleotides and the last three nucleotides in the sequence were respectively subjected to 2'-OMe modification, the first three nucleotides and the last three nucleotides were respectively linked via thiophosphate bonds, and meanwhile, the targeting base was linked to the 3' nearest base and the 5' nearest base of the targeting base respectively via thiophosphate bonds.
CM5: except the targeting base, five bases, adjacent to the targeting base, at the 5'-terminal, and five bases, adjacent to the targeting base, at the 3'-terminal, all other nucleotides were subjected to 2'-OMe modification, and meanwhile, the first three nucleotides and the last three nucleotides were respectively linked via thiophosphate bonds.
CM6, the first five nucleotides and the last five nucleotides in the sequence are respectively subjected to 2'-OMe modification, and the first five nucleotides and the last five nucleotides are respectively linked via thiophosphate bonds.
IDUA in cells was edited by transfecting GM06214 cells with different arRNAs, the cells were collected 48 h after transfection and subjected to IDUA enzymatic activity assay. As shown in Fig. 9, except the chemical modification method CM5 (the fifth modification: all nucleotides were subjected to 2'-OMe modification except 11 nt near the targeting base), the enzyme activity caused by other modification methods is high.

**Table 9**

| Name | Length | Modificatio n method | Sequence |
|---|---|---|---|
| HIV2-76-CM 1 | 55nt-c-20nt | CM1 | Gm*Am*Cm*Gro-Cro-Cro-Cro-Aro-Cro-Cro-Gro -Umo-Gro-Umo-Gro-Gro-Umo-Umo-Gro-Cro-Um o-Gro-Umo-Cro-Cro-Aro-Gro-Gro-Aro-Cro-Gro- Gro-Umo-Cro-Cro-Cro-Gro-Gro-Cro-Cro-Umo-Gr o-Cro-Gro-Aro-Cro-Aro-Cro-Umo-Umo-Cro-Gro- Gro-Cro-Cro-Cro-Aro-Gro-Aro-Gro-Cro-Umo-Gr o-Cro-Umo-Cro-Cro-Umo-Cro-Aro-Umo-Cro-Um *Gm*Cm*Gm (SEQ ID NO: 26) |
| HIV2- | 55nt-c- | CM2 | Gm*Am*Cm*Gro-Cro-Cro-Cro-Aro-Cro-Cro-Gro |
| 76-CM 2 | 20nt | | -Umo-Gro-Umo-Gro-Gro-Umo-Umo-Gro-Cro-Um o-Gro-Umo-Cro-Cro-Aro-Gro-Gro-Aro-Cro-Gro- Gro-Umo-Cro-Cro-Cro-Gro-Gro-Cro-Cro-Umo-Gr o-Cro-Gro-Aro-Cro-Aro-Cro-Umo-Umo-Cro-Gro- Gro-Cro-Cro-Cro-Amo-Gro-Aro-Gro-Cro-Umo-Gr o-Cro-Umo-Cro-Cro-Umo-Cro-Aro-Umo-Cro-Um *Gm*Cm*Gm (SEQ ID NO: 26) |
| HIV2-76-CM 3 | 55nt-c-20nt | CM3 | Gm*Am*Cm*Gro-Cro-Cro-Cro-Aro-Cro-Cro-Gro -Umo-Gro-Umo-Gro-Gro-Umo-Umo-Gro-Cro-Um o-Gro-Umo-Cro-Cro-Aro-Gro-Gro-Aro-Cro-Gro- Gro-Umo-Cro-Cro-Cro-Gro-Gro-Cro-Cro-Umo-Gr o-Cro-Gro-Aro-Cro-Aro-Cro-Umo-Umo-Cro-Gro- Gro-Cro-Cmo-Cro-Aro-Gro-Aro-Gro-Cro-Umo-Gr o-Cro-Umo-Cro-Cro-Umo-Cro-Aro-Umo-Cro-Um *Gm*Cm*Gm (SEQ ID NO: 26) |
| HIV2-76-CM 4 | 55nt-c-20nt | CM4 | Gm*Am*Cm*Gro-Cro-Cro-Cro-Aro-Cro-Cro-Gro -Umo-Gro-Umo-Gro-Gro-Umo-Umo-Gro-Cro-Um o-Gro-Umo-Cro-Cro-Aro-Gro-Gro-Aro-Cro-Gro- Gro-Umo-Cro-Cro-Cro-Gro-Gro-Cro-Cro-Umo-Gr o-Cro-Gro-Aro-Cro-Aro-Cro-Umo-Umo-Cro-Gro- Gro-Cro-Cr*Cr*Ar-Gro-Aro-Gro-Cro-Umo-Gro-C ro-Umo-Cro-Cro-Umo-Cro-Aro-Umo-Cro-Um*G m*Cm*Gm (SEQ ID NO 26) |
| HIV2-76-CM 5 | 55nt-c-20nt | CM5 | Gm*Am*Cm*Gmo-Cmo-Cmo-Cmo-Amo-Cmo-C mo-Gmo-Umo-Gmo-Umo-Gmo-Gmo-Umo-Umo- Gmo-Cmo-Umo-Gmo-Umo-Cmo-Cmo-Amo-Gmo -Gmo-Amo-Cmo-Gmo-Gmo-Umo-Cmo-Cmo-Cm o-Gmo-Gmo-Cmo-Cmo-Umo-Gmo-Cmo-Gmo-A mo-Cmo-Amo-Cmo-Umo-Umo-Cro-Gro-Gro-Cro- Cro-Cro-Aro-Gro-Aro-Gro-Cro-Umo-Gmo-Cmo- Umo-Cmo-Cmo-Umo-Cmo-Amo-Umo-Cmo-Um* Gm*Cm*Gm (SEQ ID NO 26) |
| HIV2-76-CM 6 | 55nt-c-20nt | CM6 | Gm*Am*Cm*Gm*Cm*Cro-Cro-Aro-Cro-Cro-Gr o-Uro-Gro-Uro-Gro-Gro-Uro-Uro-Gro-Cro-Uro-G ro-Uro-Cro-Cro-Aro-Gro-Gro-Aro-Cro-Gro-Gro-U ro-Cro-Cro-Cro-Gro-Gro-Cro-Cro-Uro-Gro-Cro-G ro-Aro-Cro-Aro-Cro-Uro-Uro-Cro-Gro-Gro-Cro-C ro-Cro-Aro-Gro-Aro-Gro-Cro-Uro-Gro-Cro-Uro-C ro-Cro-Uro-Cro-Aro-Ur*Cm*Um*Gm*Cm*Gm (SEQ ID NO 26) |
| HIV2-71-CM 1 | 55nt-c-15nt | CM1 | Gm*Am*Cm*Gro-Cro-Cro-Cro-Aro-Cro-Cro-Gro -Umo-Gro-Umo-Gro-Gro-Umo-Umo-Gro-Cro-Um o-Gro-Umo-Cro-Cro-Aro-Gro-Gro-Aro-Cro-Gro- Gro-Umo-Cro-Cro-Cro-Gro-Gro-Cro-Cro-Umo-Gr |
| | | | o-Cro-Gro-Aro-Cro-Aro-Cro-Umo-Umo-Cro-Gro- Gro-Cro-Cro-Cro-Aro-Gro-Aro-Gro-Cro-Umo-Gr o-Cro-Umo-Cro-Cro-Um*Cm*Am*Um (SEQ ID NO: 15) |
| HIV2-71-CM 2 | 55nt-c-15nt | CM2 | Gm*Am*Cm*Gro-Cro-Cro-Cro-Aro-Cro-Cro-Gro -Umo-Gro-Umo-Gro-Gro-Umo-Umo-Gro-Cro-Um o-Gro-Umo-Cro-Cro-Aro-Gro-Gro-Aro-Cro-Gro- Gro-Umo-Cro-Cro-Cro-Gro-Gro-Cro-Cro-Umo-Gr o-Cro-Gro-Aro-Cro-Aro-Cro-Umo-Umo-Cro-Gro- Gro-Cro-Cro-Cro-Amo-Gro-Aro-Gro-Cro-Umo-Gr o-Cro-Umo-Cro-Cro-Um*Cm*Am*Um |
| HIV2-71-CM 3 | 55nt-c-15nt | CM3 | Gm*Am*Cm*Gro-Cro-Cro-Cro-Aro-Cro-Cro-Gro -Umo-Gro-Umo-Gro-Gro-Umo-Umo-Gro-Cro-Um o-Gro-Umo-Cro-Cro-Aro-Gro-Gro-Aro-Cro-Gro- Gro-Umo-Cro-Cro-Cro-Gro-Gro-Cro-Cro-Umo-Gr o-Cro-Gro-Aro-Cro-Aro-Cro-Umo-Umo-Cro-Gro- Gro-Cro-Cmo-Cro-Aro-Gro-Aro-Gro-Cro-Umo-Gr o-Cro-Umo-Cro-Cro-Um*Cm*Am*Um (SEQ ID NO: 15) |
| HIV2-71-CM 4 | 55nt-c-15nt | CM4 | Gm*Am*Cm*Gro-Cro-Cro-Cro-Aro-Cro-Cro-Gro -Umo-Gro-Umo-Gro-Gro-Umo-Umo-Gro-Cro-Um o-Gro-Umo-Cro-Cro-Aro-Gro-Gro-Aro-Cro-Gro- Gro-Umo-Cro-Cro-Cro-Gro-Gro-Cro-Cro-Umo-Gr o-Cro-Gro-Aro-Cro-Aro-Cro-Umo-Umo-Cro-Gro- Gro-Cro-Cr*Cr*Ar-Gro-Aro-Gro-Cro-Umo-Gro-C ro-Umo-Cro-Cro-Um*Cm*Am*Um (SEQ ID NO: 15) |
| HIV2-71-CM 5 | 55nt-c-15nt | CM5 | Gm*Am*Cm*Gmo-Cmo-Cmo-Cmo-Amo-Cmo-C mo-Gmo-Umo-Gmo-Umo-Gmo-Gmo-Umo-Umo- Gmo-Cmo-Umo-Gmo-Umo-Cmo-Cmo-Amo-Gmo -Gmo-Amo-Cmo-Gmo-Gmo-Umo-Cmo-Cmo-Cm o-Gmo-Gmo-Cmo-Cmo-Umo-Gmo-Cmo-Gmo-A mo-Cmo-Amo-Cmo-Umo-Umo-Cro-Gro-Gro-Cro- Cro-Cro-Aro-Gro-Aro-Gro-Cro-Umo-Gmo-Cmo- Umo-Cmo-Cmo-Um*Cm*Am*Um (SEQ ID NO: 15) |
| HIV2-71-CM 6 | 55nt-c-15nt | CM6 | Gm*Am*Cm*Gm*Cm*Cro-Cro-Aro-Cro-Cro-Gr o-Uro-Gro-Uro-Gro-Gro-Uro-Uro-Gro-Cro-Uro-G ro-Uro-Cro-Cro-Aro-Gro-Gro-Aro-Cro-Gro-Gro-U ro-Cro-Cro-Cro-Gro-Gro-Cro-Cro-Uro-Gro-Cro-G ro-Aro-Cro-Aro-Cro-Uro-Uro-Cro-Gro-Gro-Cro-C ro-Cro-Aro-Gro-Aro-Gro-Cro-Uro-Gro-Cro-Uro-C ro*Cm*Um*Cm*Am*Um (SEQ ID NO: 15) |

| | | | |
|---|---|---|---|
| Note: ro refers to that nucleotides are not modified and ester bonds between the nucleotides are not modified; r^{∗} refers to that nucleotides are not modified and the nucleotides are linked via thiophosphate bonds; mo refers to that nucleotides are subjected to 2'-OMe modification and ester bonds between the nucleotides are not modified; and m^{∗} refers to that nucleotides are subjected to 2'-OMe modification and the nucleotides are linked via thiophosphate bonds. | | | |

### Example 10: Influence of chemical modifications on the editing efficiency

In the present example, three preferred arRNAs targeting a human IDUA mutation site and one preferred arRNA targeting a mouse IDUA mutation site were used and chemically modified by the method CM1. Concentration gradient experiments were performed on GM06214 cells and MSPI MEF (MSPI mouse embryo fibroblasts (MEF)) cells separated from a fetal mouse (IDUA W392X mouse, B6.129S-Iduatm^{1.1Kmke}/J) with a homozygous IDUA mutation (Wang D, Shukla C, Liu X, et al. Characterization of an MPS I-H knock-in mouse that carries a nonsense mutation analogous to the human IDUA-W402X mutation [published correction appears in Mol Genet Metab. 2010 Apr;99(4):439]. Mol Genet Metab. 2010;99(1):62-71. doi:10.1016/j.ymgme.2009.08.002).

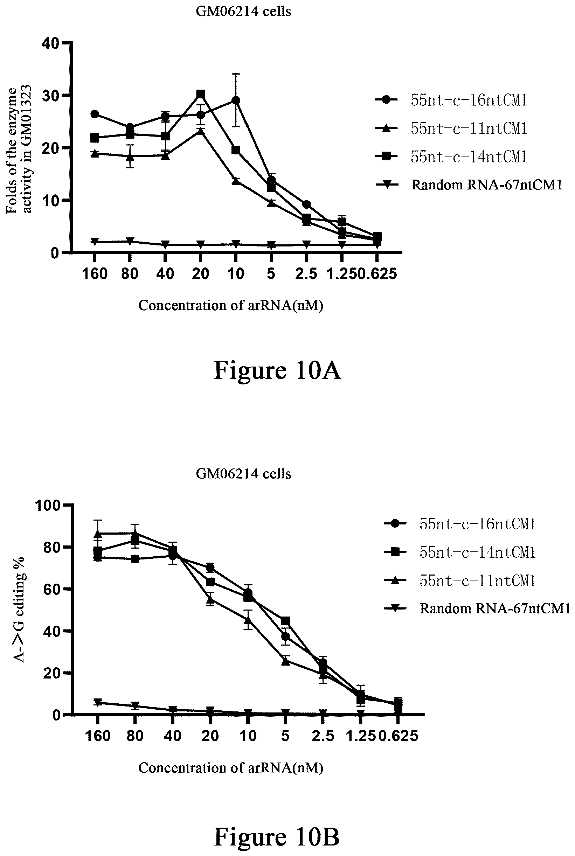

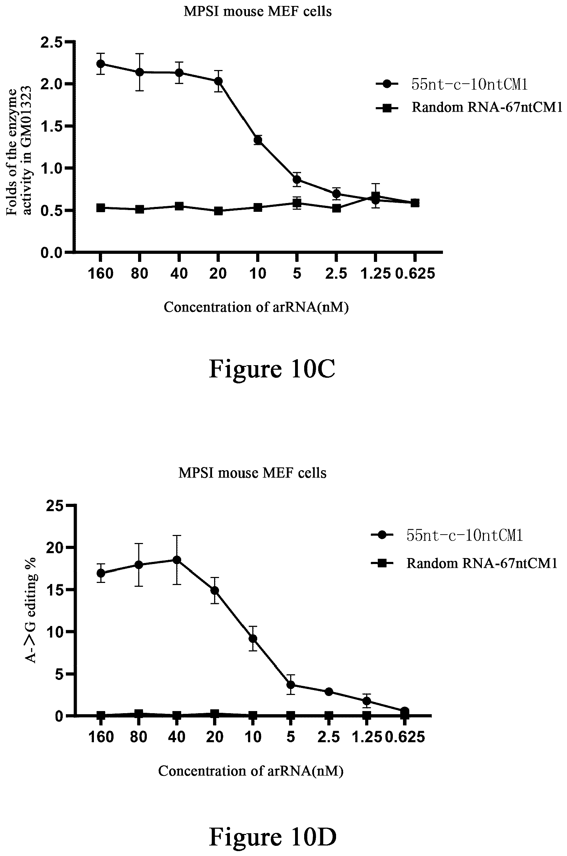

Based on the experimental results of Example 7, three arRNAs targeting the human IDUA, i.e. the sequences 55nt-c-16nt, 55nt-c-14nt, and 55nt-c-11nt, and one arRNA targeting mouse IDUA, i.e. the sequence 55nt-c-10nt, were selected. In addition, a random arRNA sequence RM-67CM1 was selected as a control. Based on the experimental results of Example 9, the above arRNAs targeting IDUA were synthesized by the chemical modification method CM1 (all bases U were subjected to 2'-OMe modification), as shown in Table 9. Comparative experiments on transfection of the human GM06214 cells and the MSPI mouse MEF with arRNAs at different concentrations were performed. Nine concentrations of arRNA were set: 160 nM, 80 nM, 40 nM, 20 nM, 10 nM, 5 nM, 2.5 nM, 1.25 nM, and 0.625 nM. The cells were coated onto a 6-well plate, transfected 24 h after coating, and digested 48 h after transfection, half of the cells were taken for IDUA enzymatic activity assay, and half of the cells were taken for RNA extraction and editing efficiency detection. As shown in Fig. 10A and Fig. 10C, if the transfection concentration of arRNA is equal to or greater than 2.5-5 nM, high enzymatic activity can be achieved, and if the transfection concentration is equal to or greater than 10-20 nM, the enzymatic activity reaches a plateau. As shown in Fig. 10B and Fig. 10D, under the condition of the same transfection concentration of arRNA, the IDUA enzymatic activity and the editing efficiency in human cells (GM06214) are different from those in mouse cells (MSPI MEF).

**Table 10**

| Name and length | Modification method | Sequence |
|---|---|---|
| 55nt-c-11nt CM1 | CM1 | `-Gm*Am*Cm*Gro-Cro-Cro-Aro-Cro-Cro-Gro-Umo- Gro-Umo-Gro-Gro-Umo-Umo-Gro-Cro-Umo-Gro-Umo-Cr o-Cro-Aro-Gro-Gro-Aro-Cro-Gro-Gro-Umo-Cro-Cro-Cro- Gro-Gro-Cro-Cro-Umo-Gro-Cro-Gro-Aro-Cro-Aro-Cro-U mo-Umo-Cro-Gro-Gro-Cro-Cro-Cro-Aro-Gro-Aro-Gro-Cro -Umo-Gro-Cr*Um*Cm*Cm (SEQ ID NO: 34) |
| 55nt-c-16nt CM1 | CM1 | `-Gm*Am*Cm*Gro-Cro-Cro-Aro-Cro-Cro-Gro-Umo- Gro-Umo-Gro-Gro-Umo-Umo-Gro-Cro-Umo-Gro-Umo-Cr o-Cro-Aro-Gro-Gro-Aro-Cro-Gro-Gro-Umo-Cro-Cro-Cro- Gro-Gro-Cro-Cro-Umo-Gro-Cro-Gro-Aro-Cro-Aro-Cro-U mo-Umo-Cro-Gro-Gro-Cro-Cro-Cro-Aro-Gro-Aro-Gro-Cro -Umo-Gro-Cro-Umo-Cro-Cr*Um*Cm*Am (SEQ ID NO: 30) |
| 55nt-c-14nt CM1 | CM1 | `-Gm*Am*Cm*Gro-Cro-Cro-Cro-Aro-Cro-Cro-Gro-Umo- Gro-Umo-Gro-Gro-Umo-Umo-Gro-Cro-Umo-Gro-Umo-Cr o-Cro-Aro-Gro-Gro-Aro-Cro-Gro-Gro-Umo-Cro-Cro-Cro- Gro-Gro-Cro-Cro-Umo-Gro-Cro-Gro-Aro-Cro-Aro-Cro-U mo-Umo-Cro-Gro-Gro-Cro-Cro-Cro-Aro-Gro-Aro-Gro-Cro -Umo-Gro-Cro-Umo-Cro-Cro-Umo-Cr*Am*Um*Cm (SEQ ID NO: 31) |
| Random RNA--67nt CM1 | CM1 | `-Um*Am*Cm*Cro-Gro-Cro-Umo-Aro-Cro-Aro-Gro-Cro- Cro-Aro-Cro-Gro-Cro-Umo-Gro-Aro-Umo-Umo-Umo-Cro- Aro-Gro-Cro-Umo-Aro-Umo-Aro-Cro-Cro-Umo-Gro-Cro- Cro-Cro-Gro-Gro-Umo-Aro-Umo-Aro-Aro-Aro-Gro-Gro-G ro-Aro-Cro-Gro-Umo-Umo-Cro-Aro-Cro-Aro-Cro-Cro-Gro -Cro-Gr*Am*Um*Gm (SEQ ID NO: 53) |
| Mouse 55nt-c-10nt CM1 | CM1 | Gm*Am*Cm*Aro-Cro-Cro-Cro-Aro-Cro-Umo-Gro-Umo- Aro-Umo-Gro-Aro-Umo-Umo-Gro-Cro-Umo-Gro-Umo-Cr o-Cro-Aro-Aro-Cro-Aro-Cro-Aro-Gro-Cro-Cro-Cro-Cro-Ar o-Gro-Cro-Cro-Umo-Umo-Umo-Gro-Aro-Gro-Aro-Cro-Cr o-Umo-Cro-Umo-Gro-Cro-Cro-Cro-Aro-Gro-Aro-Gro-Um o-Umo-Gr*Um*Um*Cmo (SEQ ID NO: 52) |

| | | |
|---|---|---|
| Note: ro refers to that nucleotides are not modified and ester bonds between the nucleotides are not modified; r^{∗} refers to that nucleotides are not modified and the nucleotides are linked via thiophosphate bonds; mo refers to that nucleotides are subjected to 2'-OMe modification and ester bonds between the nucleotides are not modified; and m^{∗} refers to that nucleotides are subjected to 2'-OMe modification and the nucleotides are linked via thiophosphate bonds. | | |

### Example 11: Sustained proteinase activity of IDUA after being edited by arRNA

In the present example, three preferred arRNAs targeting a human IDUA mutation site and one preferred arRNA targeting a mouse IDUA mutation site were used and chemically modified by the method CM1. The IDUA enzymatic activity in GM06214 cells and MSPI MEF (mouse embryo fibroblast) cells was significantly improved, which lasted for about 3 weeks.

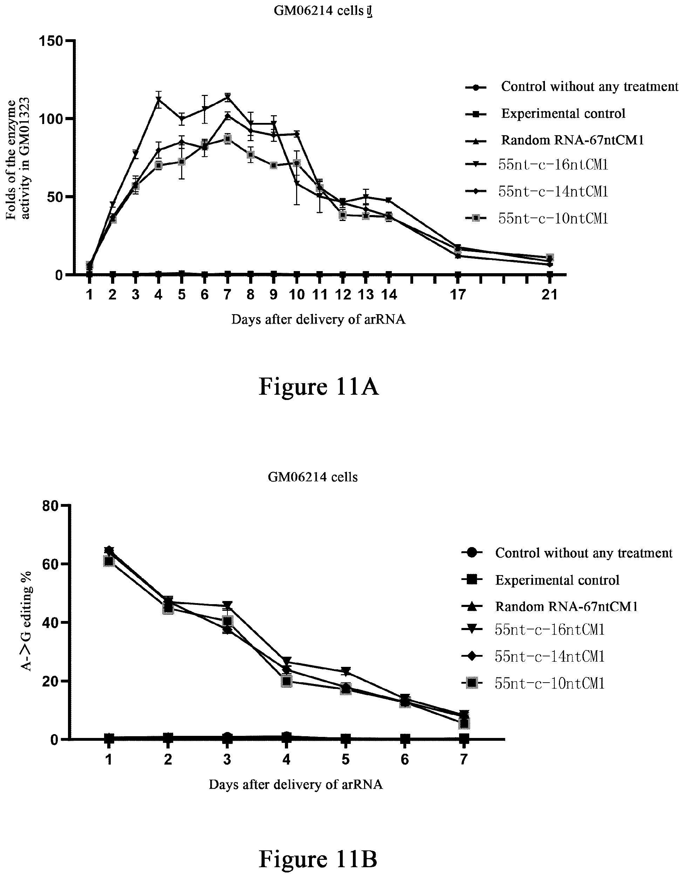

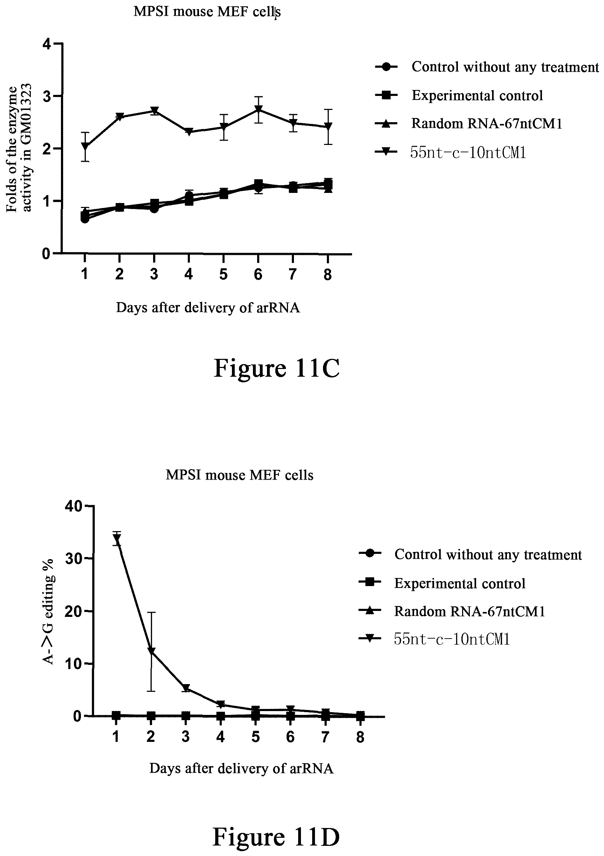

In Example 10, 48 h after transfection of the human and mouse cells with the preferred arRNAs targeting IDUA at different concentrations, the IDUA enzymatic activities were compared. In the present example, arRNA at the concentration of 20 nM was used, the IDUA enzymatic activity and the editing efficiency at different time points were compared. After transfection of GM06214 cells with arRNA, the IDUA enzymatic activity was assayed for continuous 14 d. As shown in Fig. 11A, the peak of the IDUA enzymatic activity appears on day 4 and day 9 after transfection, the IDUA enzymatic activity on day 14 is still higher than that on day 2. Then, the IDUA enzyme activity was assayed on day 17 and day 21 after transfection. As shown in Fig. 10A, the enzyme activity on day 21 is still higher than that on day 1 after transfection, and the enzyme activity is about 6 to 10 times that in GM01323. (The editing efficiency is to be added, see Fig. 11B). After transfection of MSPI MEF cells with arRNA, the IDUA enzyme activity was assayed for continuous 8 days. As shown in Fig. 11C, from 24 h to day 8 after transfection with arRNA, the enzyme activity is about 2 times that in GM1323 cells. As shown in Fig. 11D, the peak of the IDUA editing efficiency appears 24 h after transfection, and then the IDUA editing efficiency continuously decreases.

By comparison of the data of the human and mouse cells, it is found that the peak of the editing efficiency of the arRNA in the mouse cells appears 24 h after transfection, while the peak of the editing efficiency of the arRNA in the human cells appears 48 h after transfection. After editing, the IDUA enzyme activity in the human cells lasts for more than 21 days, while the IDUA enzyme activity in the mouse cells lasts for more than 8 days.

### Example 12: Influence of arRNA delivery methods on the editing efficiency

In the present example, wild-type sites on the PPIB gene in human and mouse liver cells obtained by primary cell culture were edited by LEAPER, and arRNAs were delivered by different methods to screen out the optimal delivery method.

"PPIB" refers to a wild-type site in the human NM_000942 (PPIB Genomic chr15 (-): 64163082) UTR region or a wild-type site in the mouse NM_011149 (PPIB Genomic chr9 (+): 66066490) UTR region. PPIB may be mature mRNA or precursor mRNA. The UTR region of PPIB comprises one TAG. In the present example, A in the TAG serving as a target was edited to detect the editing efficiency of arRNA of the present application in liver cells.

arRNA (55nt-c-15nt) targeting the PPIB UTR region was designed and synthesized, as shown in Table 11. A part of the synthesized arRNA was dissolved, subpackaged, stored at -80°C, and used for transfection of cells by Lipo (lipofectmine RNAiMAX), and the rest part of the arRNA was prepared into LNP. LNP were prepared with reference to Witzigmann D, Kulkarni J A, Leung J, et al. Lipid nanoparticle technology for therapeutic gene regulation in the liver[J]. Advanced Drug Delivery Reviews, 2020.; Kauffman K J, Dorkin J R, Yang J H, et al. Optimization of lipid nanoparticle formulations for mRNA delivery in vivo with fractional factorial and definitive screening designs[J]. Nano letters, 2015, 15(11): 7300-7306.; and Reis J, Kanagaraj S, Fonseca A, et al. In vitro studies of multiwalled carbon nanotube/ultrahigh molecular weight polyethylene nanocomposites with osteoblast-like MG63 cells[J]. Brazilian Journal of Medical and Biological Research, 2010, 43(5): 476-482.

Primary human liver cells were purchased from LONZA (catalog No. HUCPI), and recovered and cultured according to an operation manual (recovery medium catalog No.: MCHT50, planking medium catalog No.: MP100). After the cells adhered to walls, the medium was replaced with a hepatocyte maintenance medium 5C (referring to Xiang C, Du Y, Meng G, et al. Long-term functional maintenance of primary human hepatocytes in vitro[J]. Science, 2019, 364(6438): 399-402).

Primary mouse liver cells were separated from a C57BJ mouse (referring to Charni-Natan M, Goldstein I. Protocol for Primary Mouse Hepatocyte Isolation[J]. STAR protocols, 2020, 1(2): 100086.), and after the separated liver cells adhered to walls, the medium was replaced with a maintenance medium 5C.

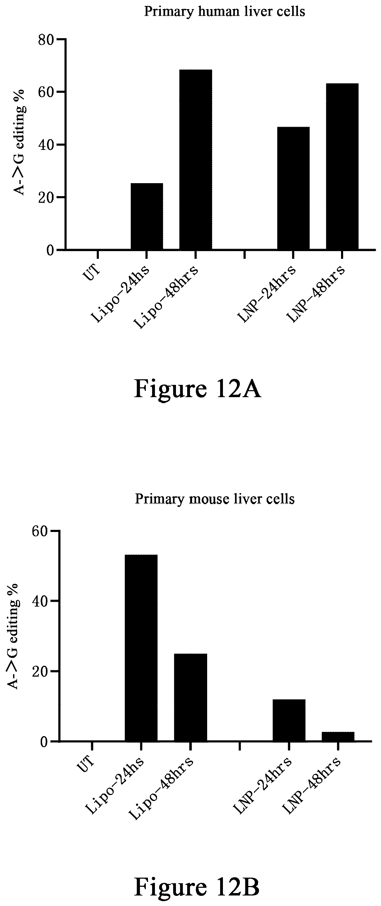

24 h after recovery of the human liver cells, the arRNA was delivered, and concentrations of arRNA delivered by LNP and Lipo were both 20 nM. 24 h after separation culture of the mouse liver cells, the arRNA was delivered, and concentrations of arRNA delivered by LNP and Lipo were both 20 nM. RNA of the human liver cells was extracted 24 h after delivery of arRNA, RNA of the mouse liver cells was extracted 48 h after delivery of arRNA, and the editing efficiency was detected by the next generation sequencing. As shown in Fig. 12A, in the human liver cells, the editing efficiency 48 h after delivery of arRNA by the two methods is higher than that 24 h after delivery of arRNA, and meanwhile, 24 h after delivery of arRNA, the editing efficiency caused by the LNP delivery is higher than that caused by the Lipo delivery, and 48 h after delivery, the editing efficiency caused by the two delivery methods is approximately the same. As shown in Fig. 12B, in the mouse liver cells, the editing efficiency 24 h after delivery of arRNA by the two methods is higher than that 48 h after delivery of arRNA, and meanwhile, 24 h and 48 h after delivery of arRNA, the editing efficiency caused by the Lipo delivery is higher than that caused by the LNP delivery.

Therefore, by comparison of the editing efficiency at the PPIB site in the primary liver cells, it is determined that the peak of editing in the mouse liver cells appears 24 h after delivery of arRNA, and the peak of editing in the human liver cells appears 48 h after delivery of arRNA, and these data coincide with the data of the human GM06214 cells and the mouse MSPI MEF cells. For delivery of arRNA to human liver cells, LNP is better than or equal to Lipo. For delivery of arRNA to mouse liver cells, Lipo is much better than LNP.

**Table 11**

| | |
|---|---|
| Mouse 55nt-c-15nt | CACCCCAUCAGAUGGAAGCACUAGGGCCAGGGUGGCACAGAACCUU GUGACUGGCCACCUUCGUCUGUGUG (SEQ ID NO: 54) |
| Human 55nt-c-15nt | GGAGGCGAAAGCAGCCCGGACAGCUGAGGCCGGAAGAGGGUGGGG CCGCGGUGGCCAGGGAGCCGGCGCCG (SEQ ID NO: 55) |

### Example 13: The editing efficiency caused by the LNP delivery

The present example involves the study on the editing efficiency of arRNA in IDUA after delivery of arRNA to human and mouse liver cells obtained by primary cell culture via LNP.

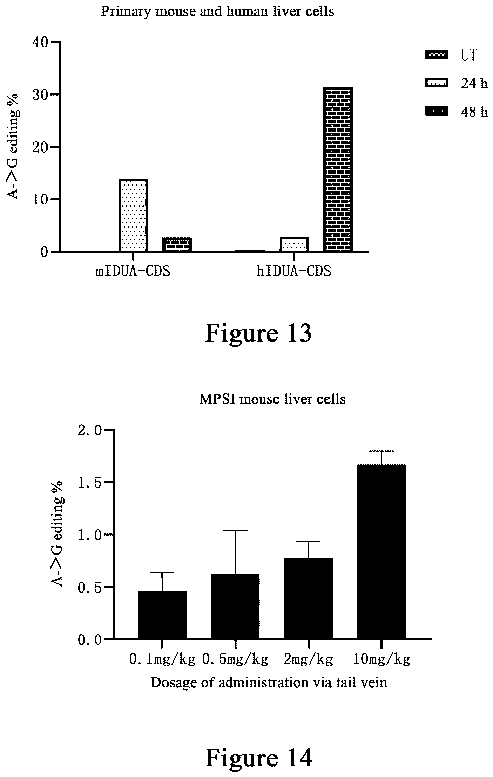

In the present example, arRNA targeting a wild-type site in the human IDUA CDS region was designed and synthesized, as shown in Table 12. The arRNAs (20 nM) targeting human and mouse IDUA were respectively delivered to primary human liver cells and primary mouse liver cells by LNP, and 24 h and 48 h after delivery, the editing efficiency was detected, As shown in Fig. 13, in the liver cells obtained by primary cell culture in vitro, the editing efficiency of arRNA in the human IDUA reaches the peak of about 30% 48 h after delivery, and the editing efficiency of arRNA in the mouse IDUA reaches the peak of about 15% 24 h after delivery. It further indicates that LNP can realize higher delivery efficiency in human cells, especially primary human liver cells.

**Table 12**

| | |
|---|---|
| Human | GGAGGCGAAAGCAGCCCGGACAGCUGAGGCCGGAAGAGGGUGGGG |
| IDUA 55nt-c-15n t | CCGCGGUGGCCAGGGAGCCGGCGCCG (SEQ ID NO: 55) |

### Example 14: Treatment effect of arRNA on IDUA in the MSPI mouse model

In the present example, an MPSI mouse model (IDUA W392X mouse, B6.129S-Iduatm^{1.1Kmke}/J) (Wang D, Shukla C, Liu X, et al. Characterization of an MPS I-H knock-in mouse that carries a nonsense mutation analogous to the human IDUA-W402X mutation [published correction appears in Mol Genet Metab. 2010 Apr;99(4): 439]. Mol Genet Metab. 2010;99(1): 62-71. Doi:10.1016/j.ymgme.2009.08.002) was used. The mutation corresponds to the human IDUA-W402X mutation, which can terminate the synthesis of proteins prematurely and is prevalent in patients with Hurler mucopolysaccharidosis (MPS I-H). Such a lysosomal storage disorder was caused by a deficiency of α-L-iduronidase. The α-L-iduronidase activity was not detected in brain and liver tissues of 5-, 10-, and 30-week-old homozygous mice. Although these homozygous mice with the mutations were viable and fertile, their average lifespan was 69 weeks. The homozygotes exhibited a progressive increase in urinary excretion of glycosaminoglycans (GAGs) and progressive accumulation of GAGs in tissues. The steady IDUA mRNA level was reduced by 30-50%. Histological analysis showed progressive accumulation of lysosomal storage inclusions in the cytoplasm of Purkinje cells and medullary neurons, and increased infiltration of foamy macrophages with age. Radiographs showed visible thickening of the zygomatic arch and femur in the 15-week-old mouse, and significant thickening in the 35-week-old mouse. At 35 weeks of age, the mineral density of the femoral bone increased, and the percentage of body fat decreased. A selected arRNA targeting a mouse IDUA mutation, i.e. 55nt-c-10nt CM1 (SEQ ID NO: 52), was prepared into LNP. arRNAs at different concentrations were injected via the tail vein, and the different concentrations were 0.1 mg/kg, 0.5 mg/kg, 2 mg/kg, and 10 mg/kg, respectively. 24 h after administration, mouse liver cells were taken for detection of the editing efficiency in IDUA. As shown in Fig. 14, 24 h after administration, the editing efficiency in the mouse injected with arRNA at the concentration of 10 mg/kg is about 2%. The results of the present example prove that the arRNA targeting IDUA in an MSPI mice model can achieve precise editing of the IDUA mutation gene in liver cells in vivo, and correct the IDUA mutation to achieve the purpose of treating MPSI.

## Claims

1. A LEAPER-based method for target editing of a target RNA in a target cell, wherein the target RNA is an RNA comprising a G-to-A mutation in an IDUA gene transcript, and the method comprises:
delivering a construct comprising an adenosine deaminase-recruiting RNA (arRNA) for editing the target RNA or a construct for encoding the arRNA to the target cell, wherein the arRNA comprises a complementary RNA sequence that hybridizes with the target RNA, and is capable of recruiting adenosine deaminase acting on RNA (ADAR) to deaminate target adenosine (A) in the target RNA.

2. The method according to claim 1, wherein the arRNA comprises base C, A, U or G that is paired with the target A.

3. The method according to any one of claims 1 to 2, wherein the length of the arRNA is about 151-61 nt, 131-66 nt, 121-66 nt, 111-66 nt, 91-66 nt or 81-66 nt.

4. The method according to claim 3, wherein a distance between a targeting base in the arRNA and the 3'-terminal is 45-5 nt, 40-5 nt, 35-10 nt, 25-15 nt or 24-11 nt.

5. The method according to claim 3 or 4, wherein the distance between the targeting base and the 5'-terminal in the arRNA is 80-30 nt, 70-35 nt, 60-40 nt, 55-35 nt or 55-45 nt.

6. The method according to any one of claims 1 to 5, wherein the target cell is a human cell.

7. The method according to any one of claims 1 to 6, wherein the target RNA is a RNA comprising the NM_000203.4(IDUA)-c.1205G-A (p.Trp402Ter) mutation site.

8. The method according to any one of claims 1 to 7, wherein the arRNA comprises any one of the following sequences: SEQ ID NO: 14, SEQ ID NO: 15, SEQ ID NO: 9, SEQ ID NO: 13, SEQ ID NO: 17, SEQ ID NO: 22, SEQ ID NO: 23, SEQ ID NO: 30, SEQ ID NO: 31, and SEQ ID NO: 34.

9. The method according to any one of claims 1 to 5, wherein the arRNA comprises any one of the following sequences: SEQ ID NO: 44 and SEQ ID NO: 52.

10. The method according to any one of claims 1 to 9, wherein the arRNA is chemically modified.

11. The method according to claim 10, wherein the chemical modification comprises 2-O'-methylation (2'-OMe) or thiophosphate modification.

12. The method according to claim 11, wherein the chemical modification is one or more selected from the group consisting of:
2'-OMe modification on each of the first three nucleotides and the last three nucleotides in the arRNA sequence;
thiophosphate bond linkage between nucleotides of the first three nucleotides and the last three nucleotides;
2'-OMe modification on all bases U in the arRNA sequence;
2'-OMe modification on the base A which is the 3' base nearest to the targeting base;
2'-OMe modification on the base C which is the 5' base nearest to the targeting base;
thiophosphate bond linkage between the targeting base with its 3' nearest base and 5' nearest base respectively;
2'-OMe modification on each of the first five nucleotides and the last five nucleotides; and
thiophosphate bond linkage between nucleotides of the first five nucleotides and the last five nucleotides.

13. The method according to any one of claims 1 to 9, wherein the construct for encoding the arRNA is a linear nucleic acid chain, virus vector or plasmid.

14. The method according to claim 13, wherein the virus vector is an adeno-associated virus (AAV) vector or lentivirus expression vector.

15. The method according to any one of claims 1 to 14, wherein the delivery method is electrotransfection, lipofection, or lipid nanoparticle (LNP) delivery or infection.

16. The method according to claim 15, wherein the construct comprising adenosine deaminase-recruiting RNA for editing target RNA or the construct for encoding the arRNA is delivered to a target cell via LNP.

17. The method according to any one of claims 1 to 16, wherein the delivery concentration of the arRNA is equal to or greater than 2.5 nM, 5 nM, 10 nM, 15 nM or 20 nM.

18. An arRNA for LEAPER-based target editing of a target RNA in a target cell or a coding sequence of the arRNA, wherein the arRNA comprises or consists of any one of the following sequences: SEQ ID NO: 14, SEQ ID NO: 15, SEQ ID NO: 9, SEQ ID NO: 13, SEQ ID NO: 17, SEQ ID NO: 22, SEQ ID NO: 23, SEQ ID NO: 30, SEQ ID NO: 31, SEQ ID NO: 34, SEQ ID NO: 44, and SEQ ID NO: 52.

19. A plasmid, virus vector, liposome or lipid nanoparticle comprising the arRNA or the coding sequence of the arRNA according to claim 18.

20. A composition or biological product comprising the arRNA or the coding sequence of the arRNA according to claim 18, or comprising the plasmid, virus vector, liposome or lipid nanoparticle according to claim 19.

21. A method for treating MPS IH in an individual, comprising correcting a G-to-A mutation, associated with MPS IH, in a target cell of the individual by the method according to any one of claims 1 to 17.

22. The method according to claim 20, wherein the mutation is NM_000203.4(IDUA)-c.1205G-A (p.Trp402Ter) mutation.

23. The method according to claim 20 or 21, wherein the arRNA is administrated at a frequency of ≥every 21 days, ≥every 17 days, ≥every 14 days or ≥every 10 days.
